# EUROPEAN PATENT SPECIFICATION

(11) **EP 1 940 863 B1**
(45) Date of publication and mention of the grant of the patent: **05.09.2012**
(21) Application number: 06776617.0
(22) Date of filing: 04.08.2006
(51) Int. Cl.: C07J 41/00, C07J 43/00, A61K 31/57, A61K 31/58, A61P 5/44

(54) **NITROOXY DERIVATIVES OF GLUCOCORTICOIDS**
NITROOXY-DERIVATE VON GLUKOKORTIKOIDEN
NITRO-OXYDERIVES DE STEROIDES DE GLUCOCORTICOIDES

(30) Priority: 02.09.2005 EP 05019155
(43) Date of publication of application: 09.07.2008
(73) Proprietor: NICOX S.A., 06560 Sophia Antipolis - Valbonne (FR); FERRER INTERNACIONAL, S.A., 08028 Barcelona (ES)
(72) Inventor: BENEDINI, Francesca, 20097 San Donato Milanese (Milano) (IT); ONGINI, Ennio, I-20090 Segrate (Milano) (IT); GUGLIETTA, Antonio, 08750 Molins de Rei (ES); PALOP, Daniel, 08390 Mongat (ES); PRINCEP, Marta, 08028 Barcelona (ES)
(74) Representative: Barchielli, Giovanna
(86) International application number: PCT/EP2006/007746
(87) International publication number: WO 2007/025632

(56) References cited:
- EP-A- 1 336 602
- WO-A-97/34871
- WO-A-97/40836
- WO-A-97/41144
- WO-A2-03/064443
- US-A- 5 482 934
- HYUN E ET AL: "Anti-inflammatory effects of nitric oxide-releasing hydrocortisone NCX 1022, in a murine model of contact dermatitis" BRITISH JOURNAL OF PHARMACOLOGY 2004 UNITED KINGDOM, vol. 143, no. 5, 2004, pages 618-625, XP002410063 ISSN: 0007-1188
- HAMMER S ET AL: "Glucocorticoid receptor interactions with glucocorticoids: evaluation by molecular modeling and functional analysis of glucocorticoid receptor mutants" STEROIDS, BUTTERWORTH-HEINEMANN, STONEHAM, MA, US, vol. 68, no. 4, April 2003 (2003-04), pages 329-339, XP004429311 ISSN: 0039-128X
- GYSLER A ET AL: "Prednicarbate biotransformation in human foreskin keratinocytes and fibroblasts." PHARMACEUTICAL RESEARCH. JUN 1997, vol. 14, no. 6, June 1997 (1997-06), pages 793-797, XP002410064 ISSN: 0724-8741
- LANGE KATHARINA ET AL: "Cutaneous inflammation and proliferation in vitro: Differential effects and mode of action of topical glucocorticoids" SKIN PHARMACOLOGY AND APPLIED SKIN PHYSIOLOGY, vol. 13, no. 2, March 2000 (2000-03), pages 93-103, XP008072279 ISSN: 1422-2868
- WALLACE JOHN L ET AL: "Enhanced anti-inflammatory potency of a nitric oxide-releasing derivative of flunisolide: role of nuclear factor-kappaB." THE JOURNAL OF PHARMACOLOGY AND EXPERIMENTAL THERAPEUTICS SEP 2004, vol. 310, no. 3, September 2004 (2004-09), pages 1096-1102, XP002418965 ISSN: 0022-3565

## Description

### TECHNICAL FIELD

The present invention relates to new steroids nitrooxyderivatives, to topical pharmaceutical formulations thereof, and their use for treating skin diseases or disorders.

### BACKGROUND ART

Most of the skin diseases or disorders are the result of inflammation caused by inflammatory agents, such as, but not limited to, bacterial, fungal, viral, parasitic, autoimmune, allergic, hormonal and/or malignant inflammatory agents. The most common skin diseases or disorders include, but is not limited to, corticosteroid-responsive dermatosis, atopic dermatitis, inflammation, eczema, erythema, papulation, scaling, erosion, oozing, crusting, pruritis, psoriasis, epidermalysis bullosa, erythema, hidradenitis suppurative, warts, diaper rash, jock itch, ruber lichen planus.
Dermatitis and eczema result from inflammatory processes that involve the upper dermis and epidermis of the skin. When eczema develops, the keratinocytes in the epidermis distend from one another and fluid is accumulated there amongst in a process known as spongiosis.

In chronic forms of eczema or dermatitis the main change include thickening of the epidermis, which leads to itching, roughening and scaling of the skin surface. The loss of water from the skin leads to inflammation of the horny layer, which later results in cracked and sore skin. Dermatitis is further classified into contact dermatitis (allergic or non allergic), atopic dermatitis and seborrheic dermatitis. Non-allergic contact dermatitis occurs in response to skin irritants, such as acids, alkalis, oils, detergents and solvents.
Allergic contact dermatitis occurs as a result of sensitization to repeated exposure to an antigen. Allergic contact dermatitis appears in skin areas that were in direct contact with the antigen.
Atopic dermatitis, which affects mainly infants, is characterized by sensitization of the skin to a wide range of common antigens.
Seborrheic dermatitis affects the scalp and other hairy areas, the face, and flexural areas and results from yeast or bacteria induced inflammation. Most people suffer from dandruff that is a mild form of seborrheic dermatitis. Psoriasis is a dominant autosomal inherited inflammatory disease characterized by enhanced proliferation of keratinocytes which proliferation leads to formation of scaly plaques on, for example, the knees, elbows, buttocks, and which are aesthetically unpleasant and cause discomfort to the affected subject.
Skin diseases or disorders are usually treated by creams, gels or ointments containing steroidal agents and/or antibacterial agents and/or antifungal agents.
Topical corticosteroids are a powerful tool for treating skin disease.
In clinical practice, for example the use of super potent topical steroids is typically limited to only two weeks because of their use may be associated with adverse side effects such as skin atrophy, burning, itching, irritation, dryness, folliculitis, hypertrichosis, acne, hypo pigmentation, perioral dermatitis, allergic contact dermatitis, maceration of the skin, and secondary infection.

Although topical administration of corticosteroids minimizes the side-effects as compared to systemic administration, the active compounds are still absorbed into the circulation where they are systemically active. Systemic absorption of topical corticosteroids can result in reversible hypothalamic-pituitary-adrenal (HPA) axis suppression, Cushing's syndrome-like symptoms, hyperglycemia, effects on bone growth in children and on bone density in the elderly, ocular complications (cataract formation and glaucoma) and skin atrophy.
Furthermore, tachyphylaxis may result from the use of the topical steroid.

Whilst the modern glucocorticoids are very much safer than those originally introduced, it remains an object of research to produce new molecules and formulations having an improved clinical efficacy, and reduced side effects.

A variety of protocols have been developed to try to increase the efficiency and/or effectiveness of a topical agent, although thus far such protocols have met with limited success. For example, dermatological agents have been provided in a variety of topical formulations such as creams, lotions, gels and the like in attempts to increase the delivery efficiency. However, while enabling direct, localized application of the dermatological agent to a skin surface, these topical formulations have not provided a complete solution as typically only partial improvement results even with an optimal formulation, e.g., oftentimes recalcitrant skin lesions remain, and/or treatment times have not been appreciably shortened.

U.S. Pat. No. 4,335,121 discloses 6.alpha., 9.alpha.-Difluoro-17.alpha.-(1-oxopropoxy)-11.beta.-hydroxy-16.alpha.-meth yl-3-oxo-androsta-1,4-diene-17-0-carbothioic acid S-fluoromethyl ester (known by the generic name of fluticasone propionate) and derivatives thereof, these compounds have good anti-inflammatory activity, particularly on topical applications.
EP 0929565 discloses nitroxyesters of corticosteroids that among systemic uses can be used for the treatment of dermatological disorders; in particular the patent discloses nitroxyesters of corticosteroids in which the nitroxy group is covalently linked through an alkyl chain to the glucocorticoid moiety. The document reports that these nitroderivatives of steroids, after systemic administration, displayed enhanced efficacy and better systemic tolerability, such as better gastric tolerability, reduced cardiovascular side effects, compared with their parent compounds.

WO03/064443 discloses nitrooxyderivatives of corticosteroids in which the nitrooxy group is covalently linked through an aromatic or a heteroarylic ring containing linker to the glucocorticoid moiety. The document reports that these nitrooxyderivatives of steroids, after systemic administration, displayed an improved pharmacological activity and lower side effect compared to their parent compounds.

WO00/61604 discloses nitrooxyderivatives of corticosteroids in which the nitrooxy group is covalently linked through an "antioxidant moiety" to the glucocorticoid moiety, such "antioxidant moieties" are compounds capable to prevent the production of free radicals and are selected on the basis of tests described in the patent application. The document reports that these compounds can be used for the treatment of pathologies associated with an oxidative stress condition in which the corresponding parent compounds show lower activity or higher toxicity.
The above-mentioned documents do not disclose the activity of the nitrooxyderivatives of corticosteroids after topical administration and in particular do not report any information regarding the local tolerability of the compounds.
WO 97/34871 discloses nitrosated or nitrosilate steroids and their use for the treatment of respiratory disorder, in particular describe the activity in a pulmonary model of allergic asthma and lung inflammation of 9-fluoro-11β-hydroxy-16α,17α-[(1-methylethylidene)bis(oxy)]pregna-1,4-diene-3,20-dione-21(4-nitrooxy)-butanoate. The patent application does not mention the use of the compounds in treatment of skin disorders.

Hyun E. et al, British Journal of Pharmacology (2004) 143, 618-625, relates to a study of the activity of hydrocortisone 21-[4'-(nitrooxymethyl)benzoate] in a model of irritant acute dermatitis, in this study oedema formation and recruitment of leukocytes were evaluated and the results demonstrate that the compound has a higher anti-inflammatory activity than the parent compound hydrocortisone. The document does not report any information regarding the effect of the compound on the skin after a long-lasting treatment. Moreover the experimental model described by Hyun E. et al is not predictive for other dermatological disorders.

### DISCLOSURE OF THE INVENTION

The present invention solves the above-mentioned problems by providing new nitrooxyderivatives of corticosteroids having an improved pharmacologically profile, better pharmacokinetic and pharmacodynamic properties and fewer adverse side effects, in particular the compounds of the invention show an improved local tolerability, such as reduction of skin blanching and skin atrophy, a fast onset of action and an increased efficacy than the existing topical corticosteroids. In particular the nitrooxyderivatives of corticosteroids of the present invention are more effective than the parent drugs in reducing local inflammation mediated vasodilatation resulting in a reduction of oedema and of the infiltration of inflammatory mediators.
An object of the present invention is compounds of general formula (I)

R-Z-X-ONO₂ (I)

wherein R is the corticosteroid residue of formula (II): 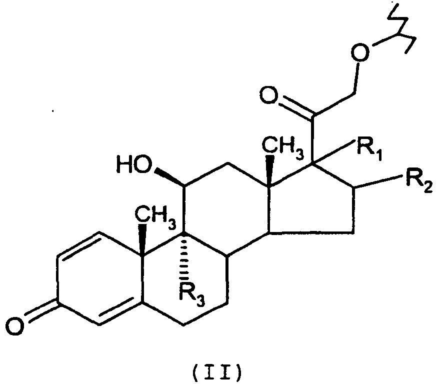 wherein
R₁ and R₂ taken together are the group of formula (III) 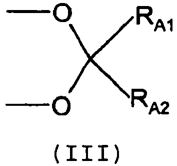 wherein
R_{A1} and R_{A2} are -CH₃ and the group of formula (III) is a isopropylidenedioxy;
R₁ and R₂ are linked to the carbon atoms in 16 and 17 of the steroidal structure in position α;
R₃ is a fluorine atom;
Z is a group capable of binding X selected from the group consisting of:
-C(O)-, -C(O)O- or 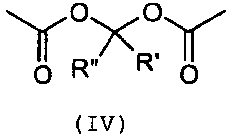 wherein R' and R" are independently selected from H or straight or branched C₁-C₄ alkyl; preferably Z is -C(O)- or
-C(O)O-;
X is a bivalent radical having the following meanings:
a) straight or branched C₁-C₂₀ alkylene, preferably a straight or branched C₁-C₁₀ alkylene, being optionally substituted with one or more of the substituents selected from the group consisting of: halogen atoms, hydroxy, -ONO₂ or T, wherein T is -OC(O)(C₁-C₁₀ alkyl)-ONO₂ or -O(C₁-C₁₀ alkyl)-ONO₂; preferably X is a straight C₁-C₁₀alkylene;
b) a C₅-C₇ cycloalkylene group optionally substituted with linear or branched C₁-C₁₀ alkyl group, preferably CH₃;
c) 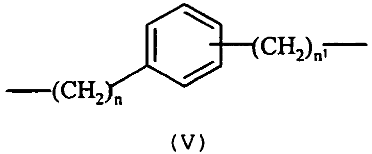
d) 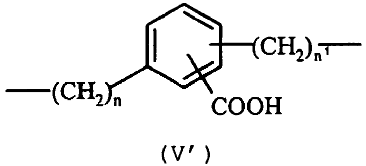 wherein n is an integer from 0 to 20, preferably n is an integer from 0 to 5; more preferably n is 0 or 1;
   n¹ is an integer from 1 to 20, preferably n¹ is an integer from 1 to 5; more preferably n¹ is 1;
e) 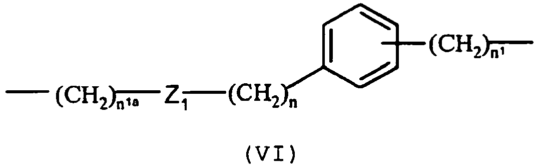 wherein n^{1a} is an integer from 1 to 20, preferably n^{1a} is an integer from 1 to 10;
   Z₁ is -C(O)O- or -OC(O)-; preferably Z₁ is -C(O)O-;
   n is as above defined;
   n¹ is as above defined;
   preferably in formula (VI) n^{1a} is an integer from 1 to 10; Z₁ is -C(O)O-, n is 0 or 1 and n¹ is 1;
   with the proviso that when X is selected from the bivalent radicals mentioned under c)-e), the -ONO₂ group of formula (I) is linked to the -(CH₂)ₙ¹- group;
f) 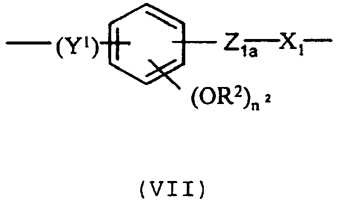 wherein:
   Y¹ is -CH₂-CH₂-(CH₂)ₙ^{2a}-, or -CH=CH-(CH₂)ₙ^{2a}- wherein and n^{2a} is an integer from 0 to 10; preferably n^{2a} is 0 or is an integer from 1 to 6;
   Z₁ₐ is -OC(O)- or -C(O)O-;
   n² is 0 or 1; preferably n² is 1;
   R² is H or CH₃; preferably R² is CH₃;
   X₁ is -(CH)ₙ^{1a}- wherein n^{1a} is as above defined, or the bivalent radical of formula (V) wherein n and n¹ are as above defined;
   preferably in formula (VII) Y¹ is -CH=CH-(CH₂)ₙ^{2a}- wherein n^{2a} is 0, Z₁ₐ is -OC(O)-, n² is 1, R² is CH₃, X₁ is -(CH)ₙ^{1a}-, wherein n^{1a} is an integer from 1 to 10;
   with the proviso that in formula (VII) the -ONO₂ group of formula (I) is linked to the X₁ group;
g) 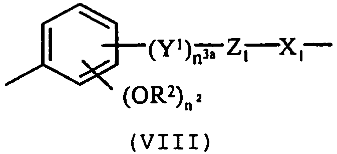 wherein:
   Y¹ is -CH₂-CH₂-(CH₂)ₙ^{2a}-, or -CH=CH-(CH₂)ₙ^{2a}- wherein and n^{2a} is an integer from 0 to 10; preferably n^{2a} is 0 or n^{2a} is an integer from 1 to 6;
   n^{3a} is 0 or 1;
   Z₁ is -C(O)O- or -OC(O)-;
   n² is 0 or 1; preferably n² is 1;
   R² is H or CH₃; preferably R² is CH₃;
   X₁ is -(CH)ₙ^{1a}- wherein n^{1a} is as above defined, or the bivalent radical of formula (V) wherein n and n¹ are as above defined;
   preferably in formula (VIII) n^{3a} is 1, Y¹ is -CH=CH-(CH₂)ₙ^{2a}- wherein n² is 0, Z₁ is -C(O)O-, n² is 1, R² is CH₃,
   X₁ is -(CH)ₙ^{1a}- wherein n^{1a} is an integer from 1 to 10; or in formula (VIII) n^{3a} is 0, Z₁ is -OC(O)- or -C(O)O-, n² is 1,
   R² is CH₃ and X₁ is -(CH)ₙ^{1a}- wherein n^{1a} is an integer from 1 to 10;
   with the proviso that in formula (VIII) the -ONO₂ group of formula (I) is linked to the X₁ group;
h) 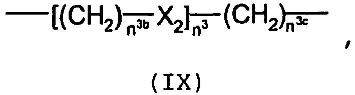 wherein
   X₂ is -O- or -S-, preferably X₂ is -O-;
   n³ is an integer from 1 to 6, preferably from 1 to 4, and
   n^{3b} is an integer from 1 to 10, preferably from 1 to 6, more preferably n^{3b} is 1 or 2;
   n^{3c} is an integer from 1 to 10, preferably from 1 to 6, more preferably n^{3C} is 2;
i) 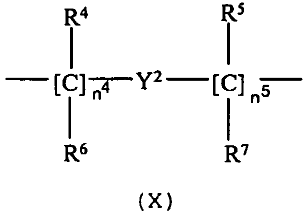 wherein:
   n⁴ is an integer from 0 to 10;
   n⁵ is an integer from 1 to 10;
   R⁴, R⁵, R⁶, R⁷ are the same or different, and are H or straight or branched C₁-C₄ alkyl, preferably R⁴, R⁵, R⁶, R⁷ are H;
   wherein the -ONO₂ group of formula (I) is linked to 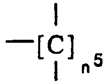
   wherein n⁵ is as defined above;
   Y² is an heterocyclic saturated, unsaturated or aromatic 5 or 6 members ring, containing one or more heteroatoms selected from nitrogen, oxygen, sulfur,
   and is selected from 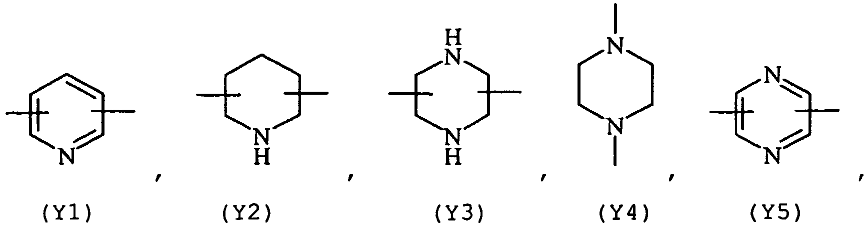 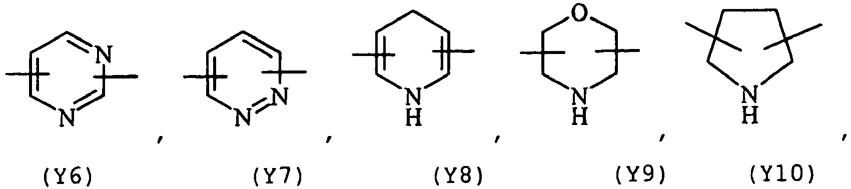 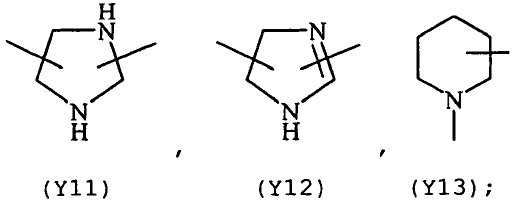
   with the proviso that when in formula (I) Z is -C(O)- then X has not the following meaning:
      a) straight or branched C₁-C₂₀ alkylene, preferably a straight or branched C₁-C₁₀ alkylene, being optionally substituted with one or more of the substituents selected from the group consisting of: halogen atoms, hydroxy, -ONO₂ or T, wherein T is -OC(O)(C₁-C₁₀ alkyl)-ONO₂ or -O(C₁-C₁₀ alkyl)-ONO₂.

Preferred bivalent radicals X are:
a) straight C₁-C₁₀ alkylene;
c) 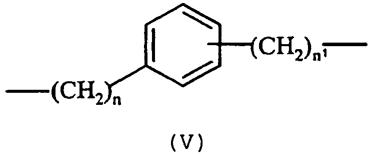 wherein n is 0 or 1 and n¹ is 1;
e) 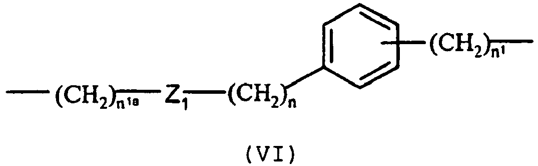 wherein n^{1a} is an integer from 1 to 10, Z₁ is -C(O)O- or - OC(O)-, n is 0 or 1 and n¹ is 1;
   with the proviso that when X is selected from the bivalent radicals mentioned under c)-e), the -ONO₂ group of formula (I) is linked to the -(CH₂)ₙ¹- group;
f) 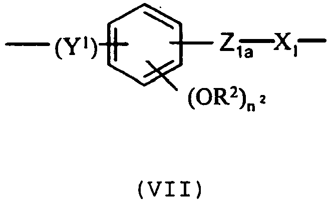 wherein:
   Y¹ is -CH=CH-(CH₂)ₙ^{2a}- wherein n^{2a} is 0, Z₁ₐ is -OC(O)-, n² is 1, R² is CH₃, X₁ is -(CH)ₙ^{1a}-, wherein n^{1a} is an integer from 1 to 10;
   with the proviso that in formula (VII) the -ONO₂ group of formula (I) is linked to the X₁ group;
g) 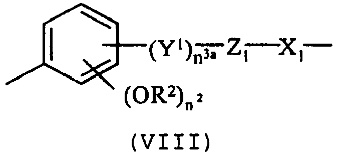 wherein:
   n^{3a} is 1, Y¹ is -CH=CH-(CH₂)ₙ^{2a}- wherein n^{2a} is 0, Z₁ is - C(O)O-, n² is 1, R² is CH₃, X₁ is -(CH)ₙ^{1a}- wherein n^{1a} is an integer from 1 to 10;
   or in formula (VIII) n^{3a} is 0, Z₁ is -OC(O)- or -C(O)O-, n² is 1, R² is CH₃ and X₁ is -(CH)ₙ^{1a}- wherein n^{1a} is an integer from 1 to 10;
   with the proviso that in formula (VIII) the -ONO₂ group of formula (I) is linked to the X₁ group;
h) 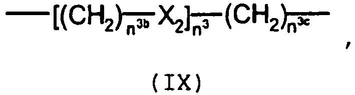 wherein
   X₂ is -O-;
   n³ is an integer from 1 to 4;
   n^{3b} is 1 or 2;
   n^{3c} is 2;

One preferred embodiment of the present invention is compounds of formula (I)

R-Z-X-ONO₂ (I)

wherein R is the corticosteroid residue of formula (II) above reported wherein:
- R₁ and R₂ both in position α are taken together and forms the group of formula (III) wherein R_{A1} and R_{A2} are -CH₃, R₃ is a fluorine atom;
Z is -C(O)- or -C(O)O-;
X has the following meanings:
a) straight C₁-C₁₀ alkylene;
c) 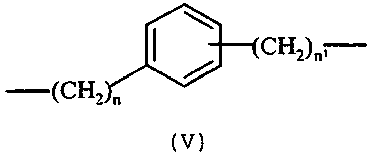 wherein n is 0 or 1 and n¹ is 1;
e) 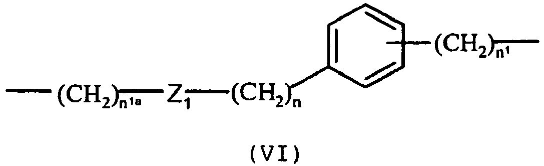 wherein n^{1a} is an integer from 1 to 10, Z₁ is -C(O)O- or - OC(O)-, n is 0 or 1 and n¹ is 1;
   with the proviso that when X is selected from the bivalent radicals mentioned under c)-e), the -ONO₂ group of formula (I) is linked to the -(CH₂)ₙ¹- group;
f) 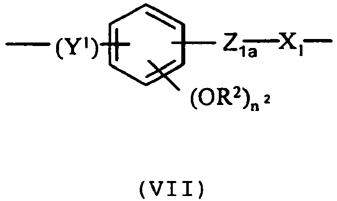 wherein:
   Y¹ is -CH=CH- (CH₂)ₙ^{2a}- wherein n^{2a} is 0, Z₁ₐ is -OC(O) -, n² is 1, R² is CH₃, X₁ is -(CH)ₙ^{1a}-, wherein n^{1a} is an integer from 1 to 10;
   with the proviso that in formula (VII) the -ONO₂ group of formula (I) is linked to the X₁ group;
g) 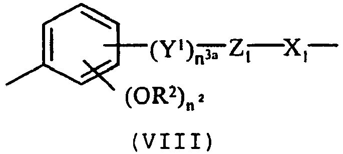 wherein:
   n^{3a} is 1, Y¹ is -CH=CH-(CH₂)ₙ^{2a}- wherein n^{2a} is 0, Z₁ is - C(O)O-, n² is 1, R² is CH₃, X₁ is -(CH)ₙ^{1a}- wherein n^{1a} is an integer from 1 to 10;
   or in formula (VIII) n^{3a} is 0, Z₁ is -OC(O)- or -C(O)O-, n² is 1, R² is CH₃ and X₁ is -(CH)ₙ^{1a}- wherein n^{1a} is an integer from 1 to 10;
   with the proviso that in formula (VIII) the -ONO₂ group of formula (I) is linked to the X₁ group;
h) 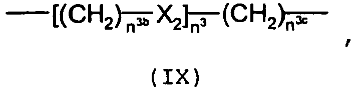 wherein
   X₂ is -O-:
   n³ is an integer from 1 to 6, preferably from 1 to 4;
   n^{3b} is 1 or 2;
   n^{3c} is 2;
   with the proviso that when in formula (I) Z is -C(O)- then X can not be straight C₁-C₁₀ alkylene;

The most preferred compounds of formula (I) of the present invention are:

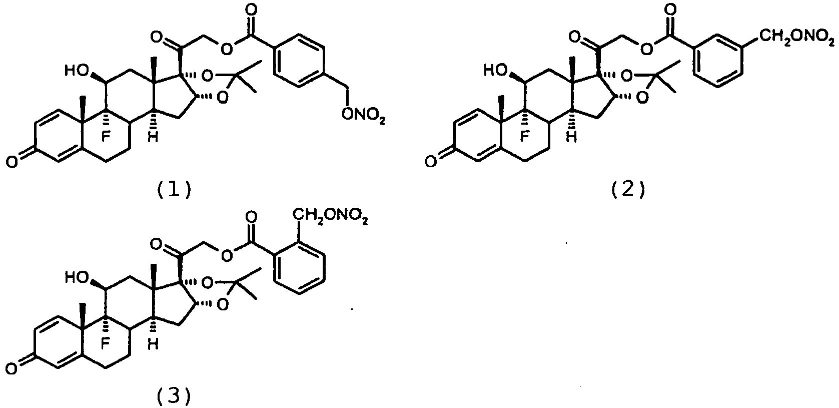

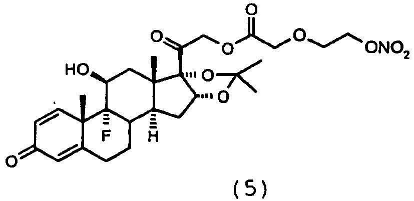

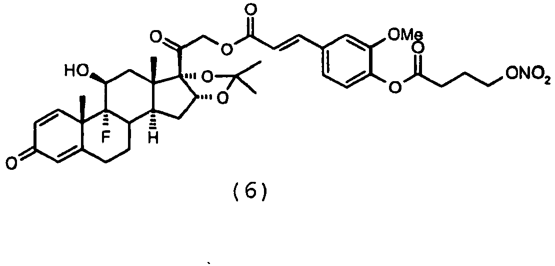

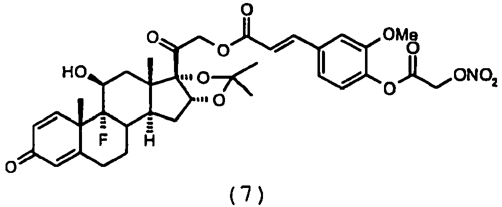

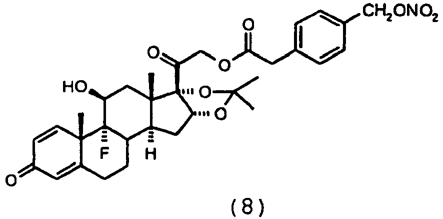

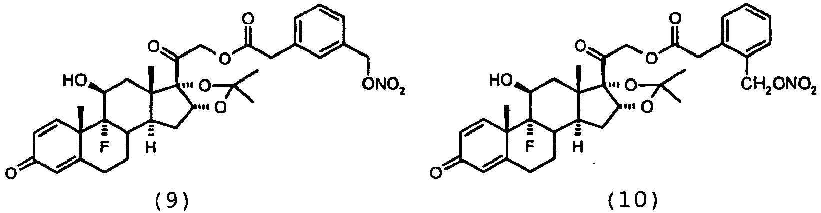

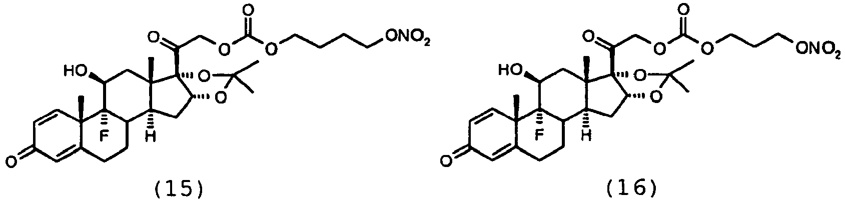

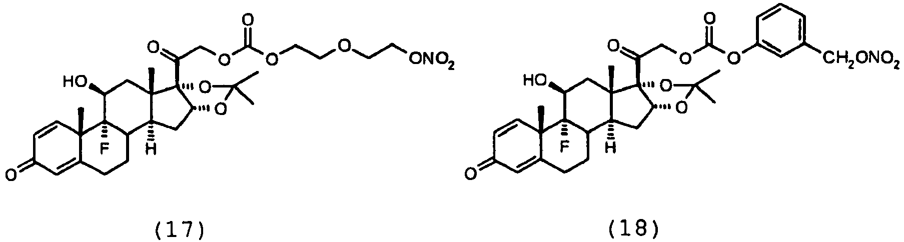

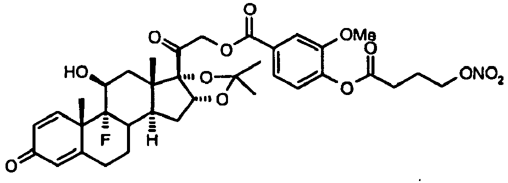

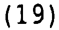

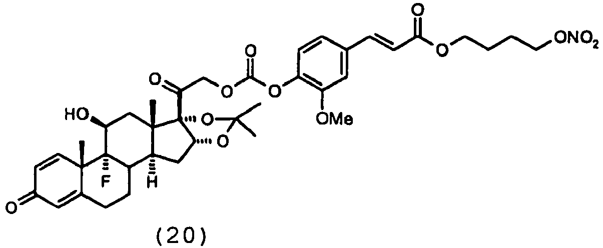

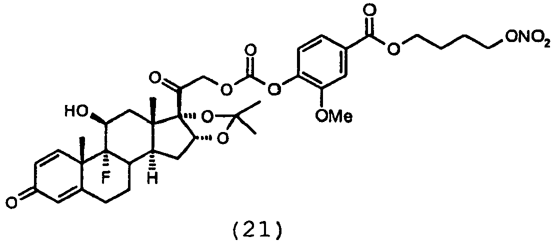

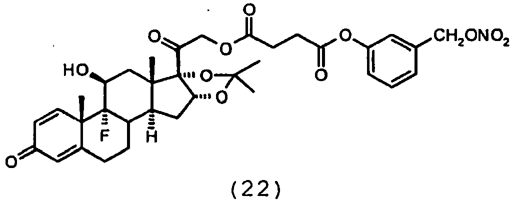

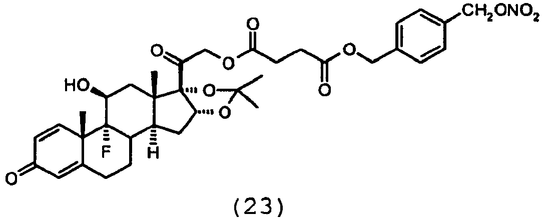

Another embodiment of the present invention is the use of compounds of formula (I)

R-Z-X-ONO₂ (I)

for treating skin diseases or disorders, wherein in formula (I):
R is the corticosteroid residue of formula (II): 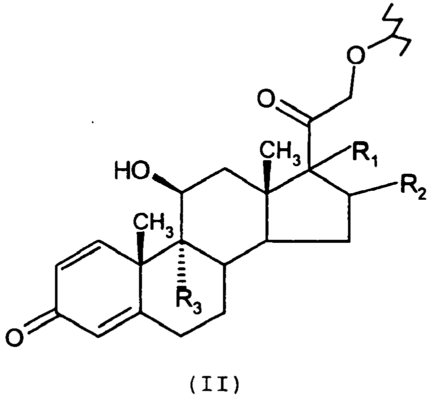 wherein
R₁ and R₂ are taken together and form the group of formula (III) 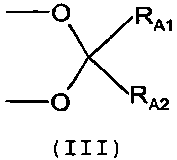 wherein R_{A1} and R_{A2} are -CH₃ and the group of formula (III) is a isopropylidenedioxy;
R₁ and R₂ are linked to the carbon atoms in 16 and 17 of the steroidal structure in position α;
R₃ is a fluorine atom;
Z is a group capable of binding X selected from the group consisting of:
   -C(O)-, -C(O)O- or 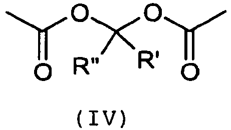 wherein R' and R" are independently selected from H or straight or branched C₁-C₄ alkyl; preferably Z is -C(O)- or
   -C(O)O-;
X is a bivalent radical having the following meanings:
   c) straight or branched C₁-C₂₀ alkylene, preferably a straight or branched C₁-C₁₀ alkylene, being optionally substituted with one or more of the substituents selected from the group consisting of: halogen atoms, hydroxy, -ONO₂ or T, wherein T is -OC(O)(C₁-C₁₀ alkyl)-ONO₂ or -O(C₁-C₁₀ alkyl)-ONO₂; preferably X is a straight C₁-C₁₀ alkylene;
   d) a C₅-C₇ cycloalkylene group optionally substituted with linear or branched C₁-C₁₀ alkyl group, preferably CH₃;
   c) 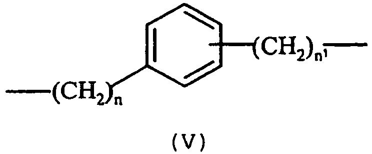
   d) 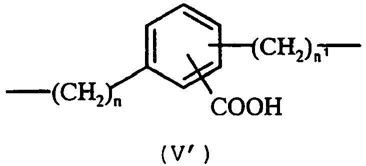 wherein n is an integer from 0 to 20, preferably n is an integer from 0 to 5; more preferably n is 0 or 1;
      n¹ is an integer from 1 to 20, preferably n¹ is an integer from 1 to 5; more preferably n¹ is 1;
   e) 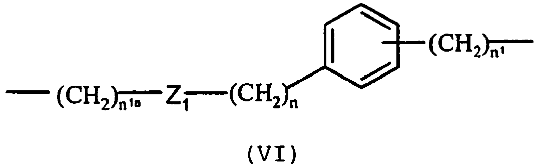 wherein n^{1a} is an integer from 1 to 20, preferably n^{1a} is an integer from 1 to 10;
      Z₁ is -C(O)O- or -OC(O)-; preferably Z₁ is -C(O)O-;
      n is as above defined;
      n¹ is as above defined;
      preferably in formula (VI) n^{1a} is an integer from 1 to 10; Z₁ is -C(O)O-, n is 0 or 1 and n¹ is 1;
      with the proviso that when X is selected from the bivalent radicals mentioned under c)-e), the -ONO₂ group of formula (I) is linked to the -(CH₂)ₙ¹- group;
   f) 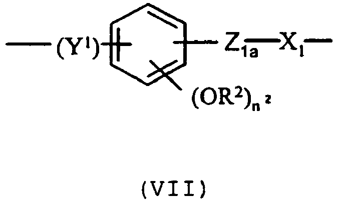 wherein:
      Y¹ is -CH₂-CH₂-(CH₂)ₙ^{2a}-, or -CH=CH-(CH₂)ₙ^{2a}- wherein and n^{2a} is an integer from 0 to 10; preferably n^{2a} is 0 or is an integer from 1 to 6;
      Z₁ₐ is -OC(O)- or -C(O)O;
      n² is 0 or 1; preferably n² is 1;
      R² is H or CH₃; preferably R² is CH₃;
      X₁ is -(CH)ₙ^{1a}- wherein n^{1a} is as above defined, or the bivalent radical of formula (V) wherein n and n¹ are as above defined;
      preferably in formula (VII) Y¹ is -CH=CH-(CH₂)ₙ^{2a}- wherein n^{2a} is 0, Z₁ₐ is -OC(O)-, n² is 1, R² is CH₃, X₁ is -(CH)ₙ^{1a}-, wherein n^{1a} is an integer from 1 to 10;
      with the proviso that in formula (VII) the -ONO₂ group of formula (I) is linked to the X₁ group;
   g) 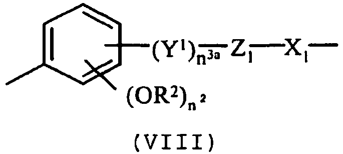 wherein:
      Y¹ is -CH₂-CH₂-(CH₂)ₙ^{2a}-, or -CH=CH-(CH₂)ₙ^{2a}- wherein and n^{2a} is an integer from 0 to 10; preferably n^{2a} is 0 or n^{2a} is an integer from 1 to 6;
      n^{3a} is 0 or 1;
      Z₁ is -C(O)O- or -OC(O)-;
      n² is 0 or 1; preferably n² is 1;
      R² is H or CH₃; preferably R² is CH₃;
      X₁ is - (CH)ₙ^{1a}- wherein n^{1a} is as above defined, or the bivalent radical of formula (V) wherein n and n¹ are as above defined;
      preferably in formula (VIII) n^{3a} is 1, Y¹ is -CH=CH-(CH₂)ₙ^{2a}- wherein n² is 0, Z₁ is -C(O)O-, n² is 1, R² is CH₃, X₁ is -(CH)ₙ^{1a}- wherein n^{1a} is an integer from 1 to 10; or in formula (VIII) n^{3a} is 0, Z₁ is -OC(O)- or -C(O)O-, n² is 1, R² is CH₃ and X₁ is -(CH)ₙ^{1a}- wherein n^{1a} is an integer from 1 to 10;
      with the proviso that in formula (VIII) the -ONO₂ group of formula (I) is linked to the X₁ group;
   h) 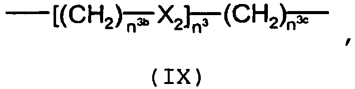 wherein
      X₂ is -O- or -S-;
      n³ is an integer from 1 to 6, preferably from 1 to 4, and n^{3b} is an integer from 1 to 10, preferably from 1 to 6, more preferably n^{3b} is 1 or 2;
      n^{3c} is an integer from 1 to 10, preferably from 1 to 6, more preferably n^{3c} is 2;
   i) 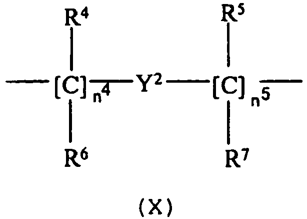 wherein:
      n⁴ is an integer from 0 to 10;
      n⁵ is an integer from 1 to 10;
      R⁴, R⁵, R⁶, R⁷ are the same or different, and are H or
      straight or branched C₁-C₄ alkyl, preferably R⁴, R⁵, R⁶, R⁷ are H;
      wherein the -ONO₂ group of formula (I) is linked to 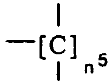
      wherein n⁵ is as defined above;
      Y² is an heterocyclic saturated, unsaturated or aromatic 5 or 6 members ring, containing one or more heteroatoms selected from nitrogen, oxygen, sulfur,
      and is selected from

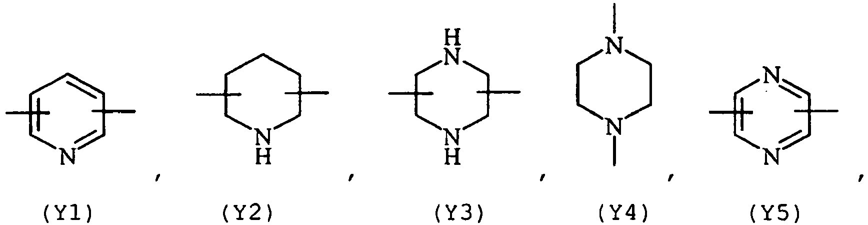

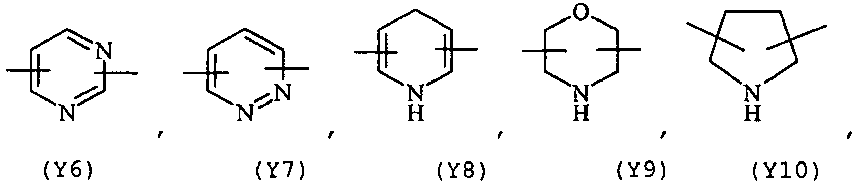

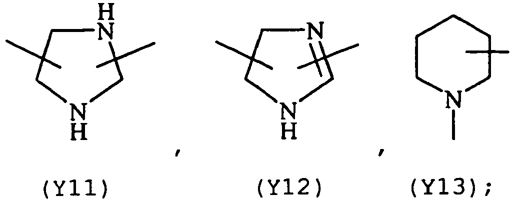

The most preferred compounds of formula (I), above reported, that can be used for treating skin diseases or disorders are:

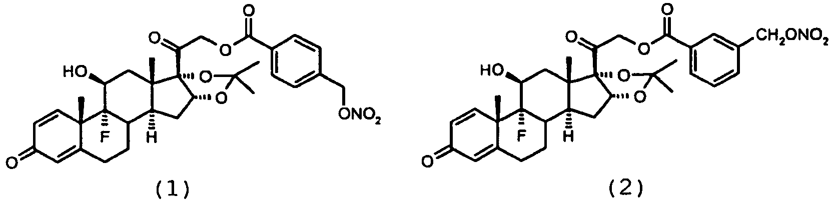

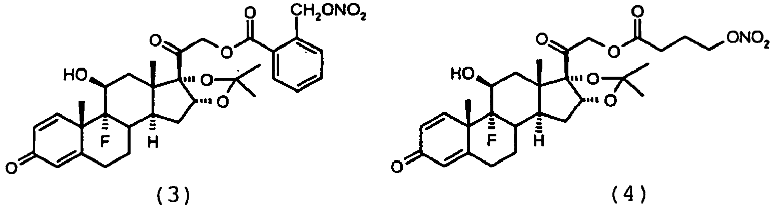

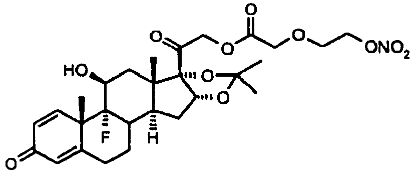

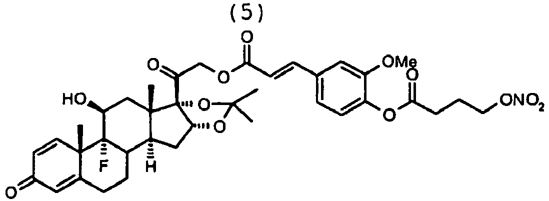

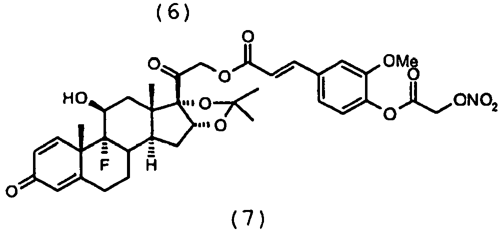

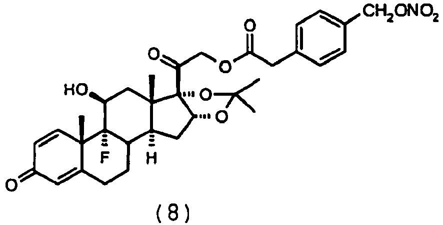

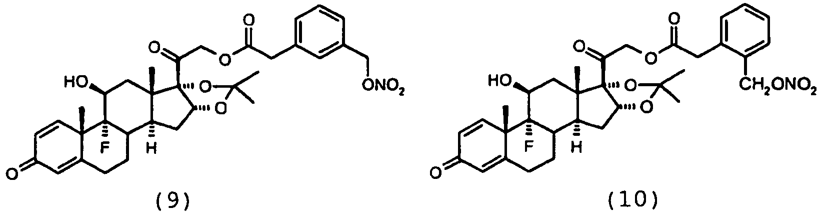

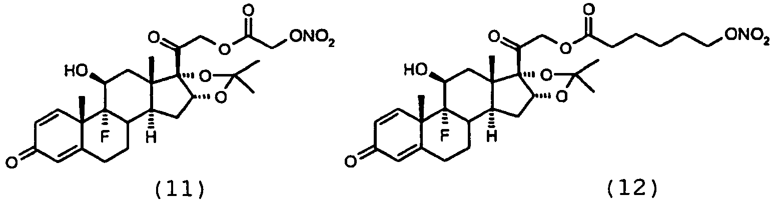

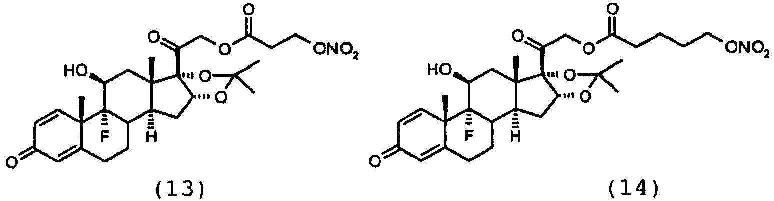

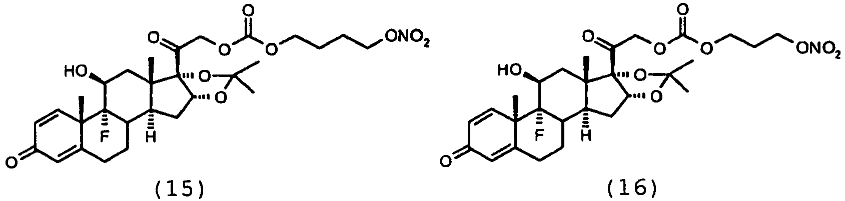

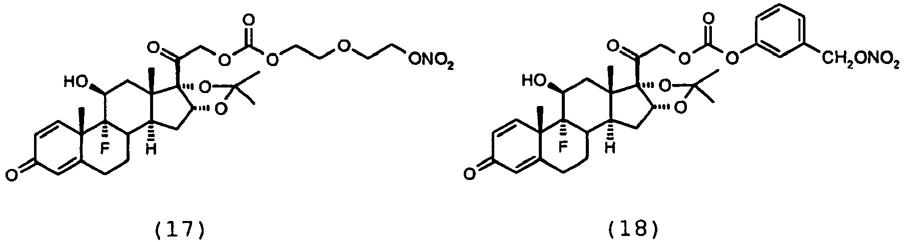

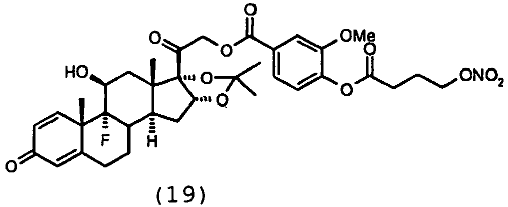

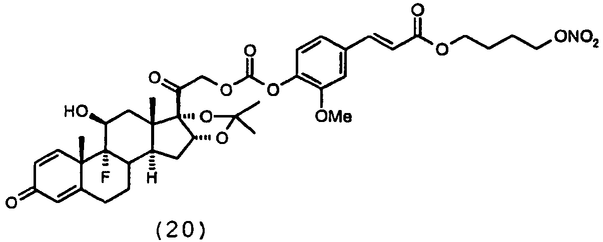

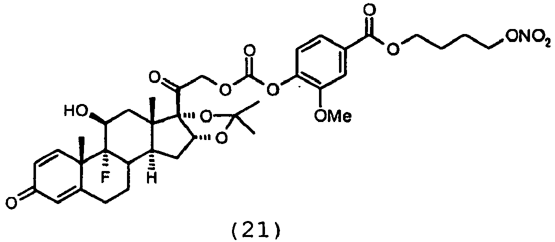

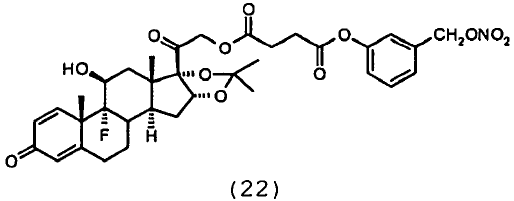

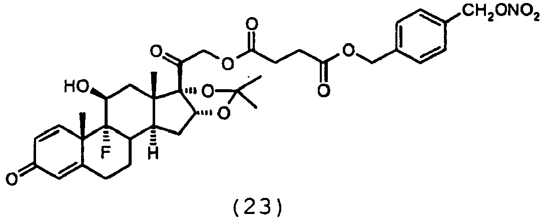

The compounds of the present invention are useful for the treatment of skin diseases or disorders comprise, but not limited, corticosteroid-responsive dermatosis, atopic dermatitis, contact dermatitis, seborrheic dermatitis, inflammation, eczema, erythema, papulation, scaling, erosion, oozing, crusting, pruritis, psoriasis, epidermalysis bullosa, erythema, hidradenitis suppurative, warts, diaper rash, jock itch, ruber lichen planus, seborrheic dermatitis which affects the scalp and other hairy areas.
The compounds of the present invention are particularly useful for the treatment of corticosteroid-responsive dermatosis, atopic dermatitis, contact dermatitis, psoriasis, seborrheic dermatitis.

The term "C₁-C₂₀ alkylene" as used herein refers to branched or straight C₁-C₂₀ hydrocarbon chain, preferably having from 1 to 10 carbon atoms such as methylene, ethylene, propylene, isopropylene, n-butylene, pentylene, n-hexylene and the like.

The term "C₁-C₁₀ alkyl" as used herein refers to branched or straight alkyl groups comprising 1 to 10 carbon atoms, including methyl, ethyl, n-propyl, isopropyl, n-butyl, isobutyl, t-butyl, pentyl, hexyl, octyl and the like.

The term "heterocyclic" as used herein refers to saturated, unsaturated or aromatic 5 or 6 members ring, containing one or more heteroatoms selected from nitrogen, oxygen, sulphur, such as for example pyridine, pyrazine, pyrimidine, pyrrolidine, morpholine, imidazole and the like.

This invention includes also the pharmaceutically acceptable salts of the compounds of formula (I).

Examples of pharmaceutically acceptable salts are either those with inorganic bases, such as sodium, potassium, calcium and aluminium hydroxides, or with organic bases, such as lysine, arginine, triethylamine, dibenzylamine, piperidine and other acceptable organic amines.

Examples of organic acids are: oxalic, tartaric, maleic, succinic, citric acids. Examples of inorganic acids are: nitric, hydrochloric, sulphuric, phosphoric acids. Salts with nitric acid are preferred.

Skin diseases or disorders comprise, but not limited, corticosteroid-responsive dermatosis, atopic dermatitis, contact dermatitis, seborrheic dermatitis, inflammation, eczema, erythema, papulation, scaling, erosion, oozing, crusting, pruritis, psoriasis, epidermalysis bullosa, erythema, hidradenitis suppurative, warts, diaper rash, jock itch, ruber lichen planus.

Also within the scope of the invention are pharmaceutical formulations suitable for topical administration comprising at least a compound of formula (I) of the present invention.
Preferred pharmaceutical dosage forms include cream, lotion and ointment formulation or topical spray compositions.
The pharmaceutical dosage forms are prepared according to procedures well known in the art.
The proportion of the active component of formula (I) in the topical formulation according to the invention depends on the precise type of formulation to be prepared but will generally be within the range of around 0.001-12% by weight, more preferably 0.001 to 10% by weight. Generally, however for most types of preparations advantageously the proportion used will be within the range of from 0.001 to 1% by weight, more preferably 0.01-0.5%, and especially around 0.025 to 0.1%.
Various optional ingredients may also be present in the topical formulations. These are: one or more various solvents such as various short chain alcohols including, but not limited to, ethyl alcohol, propylene glycol, triacetin, hexylene glycol, and combinations thereof; suitable occlusive agents that may be present in the topical formulation include, but are not limited to, petrolatum, microcrystalline wax, dimethicone, beeswax, mineral oil, squalane, liquid paraffin, shea butter, carnauba wax, SEPIGEL.RTM. (a blend of isoparaffin/polyacrylamide- /laureth-7), and combinations thereof; surfactant such as, but are not limited to, CETOMACROGOL.RTM. 1000, (Crodor, Inc.) glycerol monostearate, glycerol distearate, glyceryl stearate, polyoxyethylene stearate, a blend of glyceryl stearate and PEG-100 stearate (as ARLACEL 165), polysorbate 40, polysorbate 60, polysorbate 80, CETETH-20.RTM., sorbitan monopalimate, sorbitan monostearate, sorbitan monooleate, and combinations thereof. Other various optional ingredients may also be present in the topical formulation. These are carriers (such as water or mineral oil), skin conditioners (such as lanolin, glycerine, cholesterol, cetostearyl alcohol, dimethicone PEG 100, PEG 200, PEG 300, PEG 400 or isopropylmyristate), buffers (such as sodium citrate/citric acid, dibasic sodium phosphate/citric acid, or monobasic sodium phosphate/citric acid), or preservatives (such as imidurea, methylparaben, or propylparaben).

### EXPERIMENTAL PART

### Synthesis procedure

The compound of general formula (I) as above defined wherein Z is -CO- and X is as above defined, can be obtained
1a) by reacting a compound of formula (IIa), i.e. the precursor corticosteroid, 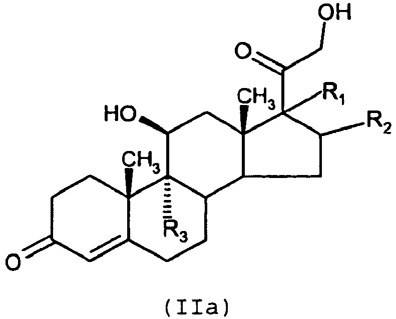 wherein R₁, R₂ and R₃ are as above defined with a compound of formula (Ib)

   W-C(O)-X-Q (Ib)

   wherein
   W is -OH, Cl, or -OC(O)Rₐ wherein Rₐ is a linear or branched C₁-C₅ alkyl or Rₐ is Rₐ₁ selected from the group consisting of: pentafluorphenoxy, 4-nitrophenoxy or succimidinyloxy;
   Q is -ONO₂ or Z₂ wherein Z₂ is selected from the group consisting of: a chlorine atom, a bromine atom, a iodine atom, a mesyl group or a tosyl group, in the presence of a condensing agent, and
1b) when Q is Z₂, by converting the compound obtained in the step a) into a nitro derivative by reaction with a nitrate source.

In step 1a) the reaction of a compound of formula (IIa) with the compound of formula (Ib) wherein W is -OH, may be carried out in presence of a condensing agent as dicyclohexylcarbodiimide (DCC), N'-(3-dimethylaminopropyl)-N-ethylcarbodiimide hydrochloride (EDAC) and a catalyst, such as N,N-dimethylamino pyridine (DMAP) or N,N'-carbonyldiimidazole (CDI). The reaction is carried out in an inert organic solvent dry such as N,N'-dimethylformamide, tetrahydrofuran, benzene, toluene, dioxane, a polyhalogenated aliphatic hydrocarbon at a temperature from -20°C and 40°C. The reaction is completed within a time range from 30 minutes to 36 hours;
In step 1a) the reaction of a compound of formula (IIa) with the compound of formula (Ib) wherein W is -OC(O)Rₐ wherein Rₐ is as above defined, may be carried out in presence of a catalyst, such as N,N-dimethylamino pyridine (DMAP) or in the presence of DMAP and a Lewis acid such as Sc(OTf)₃ or Bi(OTf)₃.

The reaction is carried out in an inert organic solvent such as N,N'-dimethylformamide, tetrahydrofuran, benzene, toluene, dioxane, a polyhalogenated aliphatic hydrocarbon at a temperature from -20°C and 40°C. The reaction is completed within a time range from 30 minutes to 36 hours. In step 1a) the reaction of a compound of formula (IIa) with the compound of formula (Ib) wherein W is Cl, X is as above defined and Q is Z₂, may be carried out in presence of an organic base such as N,N-dimethylamino pyridine (DMAP), triethylamine, pyridine. The reaction is carried out in an inert organic solvent such as N,N'-dimethylformamide, tetrahydrofuran, benzene, toluene, dioxane, a polyhalogenated aliphatic hydrocarbon at a temperature from -20°C and 40°C. The reaction is completed within a time range from 30 minutes to 36 hours.

In step 1b) the nitrate source may be silver nitrate, lithium nitrate, sodium nitrate, potassium nitrate, magnesium nitrate, calcium nitrate, iron nitrate, zinc nitrate or tetraalkylammonium nitrate (wherein alkyl is C₁-C₁₀ alkyl). The reaction is carried out in a suitable organic solvent such as acetonitrile, tetrahydrofurane, methyl ethyl ketone, ethyl acetate, DMF, in the dark, at a temperature from room temperature to the boiling temperature of the solvent. The preferred nitrate source is silver nitrate.

The compounds of formula (IIa) are commercially available or can be synthesised as described in the reference documents reported in The Merck Index - Thirteenth Edition. The compound of formula (IIa) above reported wherein R₁ and R₂ both in position α are taken together and forms the group of formula (III) wherein R_{A1} and R_{A2} are -CH₃, R₃ is a fluorine atom is known by generic name of Triamcinolone acetonide.

The compounds of formula (Ib) wherein Q is OH and X and Z₂ are as above defined, are commercially available or can be synthesized from the corresponding hydroxyl acid of formula HO-C(O)-X-OH by process well known in the art;

The compounds of formula (Ib) wherein Q is ONO₂ may be prepared from the corresponding compounds wherein Q is Z₂ by conversion to the nitro derivative as above described in step 1b).

The compounds of formula (Ib) wherein W = -OC(O)Rₐ and wherein Rₐ, X and Q are as above defined may be obtained from the corresponding acids wherein W = -OH by reaction with a chloroformate such as isobutylchloroformate, ethylchloroformate in presence of a non-nucleophilic base such as triethylamine in an inert organic solvent such as N,N'-dimethylformamide, tetrahydrofuran, a polyhalogenated aliphatic hydrocarbon at a temperature from -20°C and 40°C. The reaction is completed within a time range from 1 to 8 hours.

The compounds of formula (Ib) wherein W = Cl may be obtained from the corresponding acids wherein W = -OH by reaction with a thionyl or oxalyl chloride, halides of P^{III} or P^{v} in solvents inert such as toluene, chloroform, DMF.

The compound of general formula (I) as above defined wherein Z is -C(O)O- and X is as above defined, can be synthesised
2a) by reacting a compound of formula (IIa) above reported, with a compound of formula (Ic)

   R_{b}-C(O)O-X-Q (Ic)

   wherein X and Q are as above defined, R_{b} is Cl, Br or Rₐ₁ wherein Rₐ₁ is as above defined;
2b) when Q is Z₂, by converting the compound obtained in the step 2a) into the nitro derivative by reaction with a nitrate source as described above.

In step 2a) the reaction is generally carried out in presence of a inorganic or organic base in an aprotic polar/non-polar solvent such as DMF, THF or CH₂Cl₂ at temperatures range between 0°-65°C or in a double phase system H₂O/Et₂O at temperatures range between 20°- 40°C; or in the presence of DMAP and a Lewis acid such as Sc(OTf)₃ or Bi(OTf)₃ in solvents such as DMF, CH₂Cl₂.

The compound of formula (Ic) wherein X and Q are as above defined and R_{b} is Cl, Br may be synthesized from the corresponding alcohol of formula (Id) HO-X-Q by process well known in the art.

The compounds of formula (Ic) wherein Q is Z₂ are commercially available.

The compounds of formula (Ic) wherein Q is ONO₂ may be prepared from the corresponding compounds wherein Q is Z₁ by conversion to the nitro derivative as above described. The compound of formula (Ic) R_{b}-C(O)O-Y-Q wherein Y and Q are as above defined, R_{b} is Rₐ₁ may be obtained reacting a compound of formula (Id) HO-X-Q, with a compound of formula (Ic') R_{b}-C(O)O-Z₂ wherein Z₂ is as above defined. The reaction is generally carried out in presence of a inorganic or organic base in an aprotic polar/non-polar solvent such as DMF, THF or CH₂Cl₂ at temperatures range between 0°-65°C or in a double phase system H₂O/Et₂O at temperatures range between 20°- 40°C; or in the presence of DMAP and a Lewis acid such as Sc(OTf)₃ or Bi(OTf)₃ in solvents such as DMF, CH₂Cl₂.

The compounds of formula (Ic') wherein R_{b} is Rₐ₁ and Z₂ is as above defined are commercially available.

### EXAMPLES

### Example 1

### Synthesis of (11β,16α)-9-fluoro-11-hydroxy-16,17-[1-methyl ethylidenebis(oxy)]-21-[1-oxo-[4-(nitrooxymethyl)benzoxy]] pregna-1,4-diene-3,20-dione

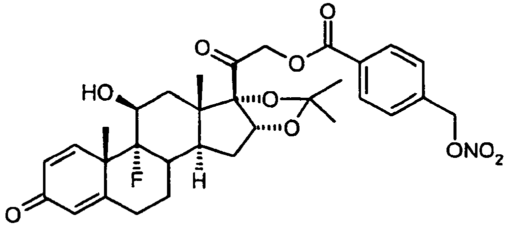

To a solution of triamcinolone acetonide (2.47 g, 5.7 mmol) in dichlorometane (55 ml), 4-(nitrooxymethyl)benzoic acid (1.38 g, 7.0 mmol), DMAP (0.07 g, 0.54 mmol) and EDAC (1.39 g, 7.2 mmol) were added. The reaction was stirred at room temperature for 24 hours. The solution was treated with water, the organic layers were dried with sodium sulfate and concentrated under reduced pressure. The residue was purified by flash chromatography, eluent dichloromethane/ethyl acetate 95/5. The product (1.2 g) was obtained as white powder.

¹H-NMR (DMSO) δ: 8.05 (2H, d); 7.64 (2H, d); 7.29 (1H, d); 6.23 (1H, dd); 6.01 (1H, s); 5.68 (2H, s); 5.52 (1H, d); 5.42 (1H, d); 5.01 (1H, d); 4.86 (1H, d); 4.2 (1H, bs); 2.7-2.25 (4H, m); 2.15-1.72 (4H, m); 1.65-1.45 (5H, m); 1.36 (3H, s); 1.21 (3H, s); 0.87 (3H, s).

### Example 2

### Synthesis of (11β,16α)-9-fluoro-11-hydroxy-16,17-[1-methylethylidenebis(oxy)]-21-[1-oxo-[3-(nitrooxymethyl) benzoxy]]pregna-1,4-diene-3,20-dione

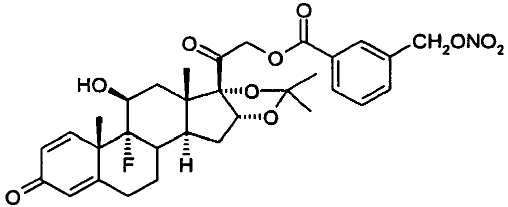

### E) (11β,16α)-9-fluoro-11-hydroxy-16,17-[1-methylethylidene bis(oxy)]-21-[1-oxo-[3-(chloromethyl)benzoxy]]pregna-1,4-diene-3,20-dione

To a solution of triamcinolone acetonide (1.5 g, 3.4 mmol) in dichlorometane (35 ml), 3-(chloromethyl)benzoic acid (0.83 g, 4.9 mmol), DMAP (0.042 g, 0.34 mmol) and EDAC (0.84 g, 4.4 mmol) were added. The reaction was stirred at room temperature for 24 hours. The solution was treated with a saturated solution of sodium bicarbonate, water, the organic layers were dried with sodium sulfate and concentrated under reduced pressure. The product (1.86 g) was obtained as white powder.

### F) (11β,16α)-9-fluoro-11-hydroxy-16,17-[1-methylethylidene bis(oxy)]-21-[1-oxo-[3-(nitrooxymethyl)benzoxy]]pregna-1,4-diene-3,20-dione

A solution of compound E (1.8 g, 3.07 mmol) and silver nitrate (1.1 g, 6.5 mol) in acetonitrile (25 ml) and tetrahydrofurane (10 ml) was stirred at 60°C, in the dark, for 18 hours. The precipitated (silver salts) was filtered off and the solvent was evaporated under vacuum. The residue was purified by flash chromatography, eluent n-hexane/ethyl acetate 65/35. The product (1.3 g) was obtained as white powder
¹H-NMR (DMSO) δ: 8.12 (1H, s); 8.03 (1H, d); 7.80 (1H, d); 7.63 (1H, t); 7.30 (1H, d); 6.23 (1H, dd); 6.01 (1H, s); 5.66 (2H, s); 5.46 (1H, bd); 5.45-5.37 (1H, m); 5.05-4.98 (1H, m); 4.86 (1H, bd); 4.22 (1H, bs); 2.72-2.29 (3H, m); 2.13-1.71 (4H, m); 1.62-1.49 (5H, m); 1.42-1.29 (4H, m); 1.2 (3H, s); 0.9 (3H, s).

### Example 3

### Synthesis of (11β,16α)-9-fluoro-11,21-dihydroxy-16,17-[1-methylethylidenebis(oxy)]pregna-1,4-diene-3,20-dione-21-[3-carboxy-1-oxopropoxy)-3-(nitrooxymethyl)benzene]

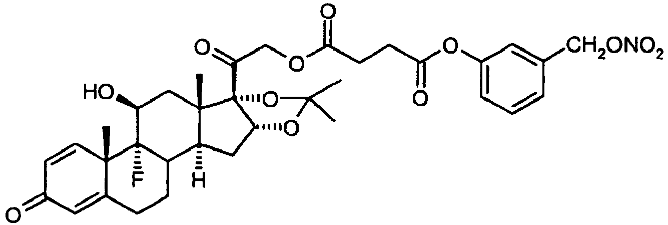

### G) (11β,16α)-9-Fluoro-11,21-dihydroxy-16,17-[1-methyl ethylidenebis(oxy)]pregna-1,4-diene-3,20-dione 21-hemisuccinate

To a solution of triamcinolone acetonide (1.0 g, 2.3 mmol) in tert-butanol (20 ml), succinic anhydride (0.72 g, 7.0 mmol) and triethylamine (0.98 ml, 7.0 mmol) were added. The reaction was stirred at room temperature for 3 hours. The solution was treated with a solution of phosphoric acid 2.5% and dichloromethane, the organic layers were dried with sodium sulfate and concentrated under reduced pressure. The product (1.33 g) was obtained as white powder H) (11β,16α)-9-Fluoro-11,21-dihydroxy-16,17-[1-methyl ethylidenebis(oxy)]pregna-1,4-diene-3,20-dione-21-[3-carboxy-1-oxopropoxy)-3-(nitrooxymethyl)benzene]

To a solution of compound obtained in step G (1.23 g, 2.3 mmol) in dichlorometane (50 ml), 3-(nitrooxymethyl)phenol (0.43 g, 2.53 mmol), DMAP (0.028 g, 0.23 mmol) and EDAC (0.56 g, 2.9 mmol) were added. The reaction was stirred at room temperature for 2 hours. The solution was treated with water, the organic layers were dried with sodium sulfate and concentrated under reduced pressure. The residue was purified by flash chromatography, eluent n-hexane/acetone 7/3. The product (1.13 g) was obtained as white powder
¹H-NMR (DMSO) δ: 7.5-7.41 (1H, m); 7.40-7.31 (1H, m); 7.30 (2H, m); 7.19-7.12 (1H, dd); 6.25-6.18 (1H, dd); 6.01 (1H, s); 5.54 (2H, s); 5.43 (1H, dd); 5.22-5.12 (1H, d); 4.85 (1H, d); 4.83-4.73 (1H, m); 4.20 (1H, bs); 2.92-2.79 (4H, m); 2.72-2.24 (4H, m); 2.15-1.72 (4H, m); 1.65-1.45 (5H, m); 1.36 (3H, s); 1.21 (3H, s); 0.87 (3H, s).

### PHARMACOLOGICAL EXAMPLES

### Example 4

### In vivo determination of inhibition of TPA-induced ear oedema after topical administration of test compounds.

The tested compounds are:
- (11β,16α)-9-fluoro-11-hydroxy-16,17-[1-methylethylidene bis(oxy)]-21-[1-oxo-[4-(nitrooxymethyl)benzoxy]] pregna-1,4-diene-3,20-dione, prepared as described in example 1;
- Triamcinolone acetonide that is the reference compound for the compound of example 1;

The tests were performed according to the methods described by Carlson et al., Agents Actions 17:197-204, 1985, and Lucas et al., J Pharmacol Exp Ther 304:1172-1180, 2003.

Groups of 5-9 male Swiss mice of 27±5 g were used. Inflammation dermatitis was induced by applying 2 µg/ear of TPA (Tetradecanoyl Phorbol Acetate) dissolved with ethanol absolute on the surface of either the dorsal aspect of both ears (20 µL/ear).

15 min before the application of TPA, mice received topically 20 µL of a solution of test compounds (0.39 nM in ethanol) per site applied directly on the skin of the left ear and the vehicle (ethanol 100%) on right ear. Vehicle-Vehicle treated mice were included as negative control group. Compounds were tested at equimolecular doses. Animals were sacrificed at 3h or 5h post TPA dose. Equal sections of both ears were punched out immediately after and weighed. The percentage of change of the weight of left ear versus the weight of right ear was calculated for each animal, and the percentage of inhibition of change in weight of treated animals versus the change in weight of non-treated animals (negative control) was measured. The results of this test are given in Table 1.

**Table 1.**

| Compound | % inhibition 3 hours post-treatment | % inhibition 5 hours post-treatment |
|---|---|---|
| Compound of Ex. 1 | 22.70 | 21.01 |
| Triamcinolone acetonide | 14.90 | 12.19 |

### Example 5

### Effects of test compounds on the 12-O-tetradecanoylphorbol acetate (TPA)-induced increase of inflammatory markers ( PGE₂ and TNF-α) in mouse ear.

The tested compounds are:
- (11β,16α)-9-fluoro-11-hydroxy-16,17-[1-methylethylidene bis(oxy)]-21-[1-oxo-[4-(nitrooxymethyl)benzoxy]] pregna-1,4-diene-3,20-dione, prepared as described in example 1;
- Triamcinolone acetonide that is the reference compound for the compound of example 1;

Ten Swiss male mice were used for treatment group. 12-*O*-Tetradecanoylphorbol acetate (2.0 µg, TPA) dissolved in 20 µl ethanol absolute was applied in 10 µl volumes to both inner and outer surfaces of the right ear of mice. A ear section of the right ear of mice were homogenized in 500 µl saline, and after centrifugation at 1,200 *g* for 15 min at 4°C, the PGE₂ and TNF-α levels were determined by radioimmunoassay (Hoult et al., Methods Enzymol 1994) or by time-resolved fluoroimmunoassay (Pennanen et al., Int J Immunopharmacol. 1995), respectively. Test compounds dissolved in the vehicle were applied topically 15 min before TPA administration. Results are reported in table 2.

**Table 2.**

| Compound | TNFalpha (pg/ml) | PGE₂ (ng/ml) |
|---|---|---|
| Vehicle + TPA | 316.2±35.5 | 97.9±6.1 |
| Compound of Example 1 (0.48 µg/ear) | 149.5±17.8** | 53.2±4.5** |
| Triamcinolone acetonide (1 µg/ear) | 158.0±24.6** | 43.0±3.8** |

| | | |
|---|---|---|
| Results show Mean±S.E.M. (n=10). ** P<0.01 with respect vehicle-TPA group | | |

Results demonstrated that at low doses, the compound of example 1 of the present invention displayed reduction of PGE₂ level than higher dose of the prior-art compound, the results show that the compound of the invention is more effective in reducing the inflammation than the correspondent parent compound.

Surprisingly, the results show that the compound of example 1 of the present invention inhibits the release of TNF-α in a higher potency than the prior art compound as displayed in table 2, therefore being more effective in reducing inflammation levels.

### Example 6

### Anti-inflammatory properties in a model of contact dermatitis induced by Benzalkonium in mice.

The tested compounds are:
- (11β,16α)-9-fluoro-11-hydroxy-16,17-[1-methylethylidene bis(oxy)]-21-[1-oxo-[4-(nitrooxymethyl)benzoxy]] pregna-1,4-diene-3,20-dione, prepared as described in example 1;
- Triamcinolone acetonide that is the reference compound for the compound of example 1 (ref. compound);

Irritant contact dermatitis was induced by applying 5% benzalkonium chloride (100 µl per site, dissolved in olive oil: acetone, 1:5 v/v) on the dorsal aspect of the two ears. Ear diameter was measured as a parameter for edema formation, before and hourly for 6 hours after benzalkonium chloride application, using an electronic calliper. The last measurement was performed at 8-hours after dermatitis induction.

The tested compounds were applied topically dissolved in ethanol:sterile water (1:1) and applied at a final volume of 100 µl, five minutes after irritant contact dermatitis will be induced.

Ear edema (left ears) value for times 1 to 4 hours after irritant stimuli with benzalconium chloride are represented in the table 3.

The compound of the present invention (comp. Ex. 1) showed a dose-dependent effect in inhibiting ear edema with a better profile than triamcinolone acetonide, mainly at earlier times.

**Table 3**

| Treatment | Time after benzalkonium application (hours) | | | |
|---|---|---|---|---|
| | 1 | 2 | 3 | 4 |
| Comp. Ex. 1 0.3 nmol | 0.026±0.005 | 0.035±0.008 | 0.053±0.009 | 0.047±0.007 |
| Comp. Ex. 1 1 nmol | 0.020±0.006 | 0.035±0.008 | 0.057±0.011 | 0.065±0.010 |
| Comp. Ex. 1 3 nmol | 0.018±0.004 | 0.042±0.007 | 0.042±0.010 | 0.070±0.007 |
| Ref. comp. 3 nmol | 0.037±0.005 | 0.063±0.010 | 0.041±0.018 | 0.068±0.022 |
| Vehicle | 0.039±0.007 | 0.052±0.008 | 0.067±0.007 | 0.079±0.007 |

### Example 7

### 4- Anti-inflammatory properties in a model of Intravital microscopy in mice

The tested compounds are:
- (11β,16α)-9-fluoro-11-hydroxy-16,17-[1-methylethylidene bis(oxy)]-21-[1-oxo-[4-(nitrooxymethyl)benzoxy]] pregna-1,4-diene-3,20-dione, prepared as described in example 1;
- Triamcinolone acetonide that is the reference compound for the compound of example 1;

C57B16 male mice will be anaesthetized by intraperitoneal injection of a mixture of 10 mg/kg xylazine (MTC Pharmaceuticals, Cambridge, Ontario, Canada) and 200 mg/kg ketamine hydrochloride (Rogar/STB, London, Ontario, Canada). Intravital microscopy will be performed on skin flap, which thickness does not allow visualizing leukocyte/endothelial cells interaction by simple transillumination. Therefore, after anaesthesia, mice will receive an intravenous injection of a fluorescent dye, rhodamine 6G (Sigma, St. Louis, MO, USA, 0.3 mg/kg). At this dose, rhodamine 6G labels leukocytes and platelets and has been shown to have no effect on leukocyte kinetics. Then, a midline abdominal incision will be performed, from the diaphragm, extending to the pelvic region. The skin will carefully be separated from the underlying tissue, but remained attached laterally, so the blood supply to the skin flap remained intact. The skin flap will be extended over a viewing pedestal to expose the dermal microvasculature and secured along the edges using 4.0 sutures. The exposed dermal tissues will be superfused with a bicarbonate-buffered saline pH 7.4, to avoid tissue dehydration. The microcirculation will be observed using an inverted microscope (Nikon) with a X20 objective lens, and rhodamine 6G allows visualization and quantification of the number of rolling and adherent leukocytes, by epi-illumination at 510-560 nm, using a 590-nm emission filter. Single unbranched venules (20-40 µm in diameter) will be selected for the study. Images of the selected venule will be recorded for-5 min, after a 15-min equilibration period and the end of this 5-min interval was considered as time 0. Leukocyte adherence will be determined upon video playback, on 100 µm vessel length (table 4). A leukocyte will be considered adherent to the endothelium if it remained stationary for 30 s or more. Leukocyte flux will be defined as the number of leukocytes per minute moving at a velocity less than that of erythrocytes, which passed a reference point in the venule. The changes in flux of rolling leukocytes will be evaluated as differences between the number of rolling leukocytes at each interval and the basal number of rolling leukocytes (table 5).

The tested compounds were applied topically dissolved in ethanol:sterile water (1:1) and applied at a final volume of 100 µl, five minutes after irritant contact dermatitis will be induced.

Results demonstrated that the compound of the invention displayed statistically significant changes in vessel diameter (Table 4), moreover, both the tested compounds reduced statistically the rolling leukocytes (Table 5), a primary inflammation endpoint, in higher degree than prior-art compounds.

**Table 4: Changes in vessel diameter**

| Treatment | Area under the curve (change in vessel diameter) |
|---|---|
| Vehicle | 546 ± 135 |
| Compound Ex. 1 (3 nmol) | 114 ± 32* |
| Triamcinolone (3 nmol) | 278 ± 78 |

| | |
|---|---|
| • Statistically different from vehicle | |

**Table 5: Rolling leukocytes**

| Treatment | Area under the curve (change in flux of rolling leucocytes) |
|---|---|
| Vehicle | 941 ± 188 |
| Compound Ex. 1 (3 nmol) | 247 ± 35* |
| Triamcinolone (3 nmol) | 376 ± 129* |

| | |
|---|---|
| * Statistically different from vehicle | |

## Claims

1. Compounds of general formula (I)
R-Z-X-ONO₂ (I)
wherein R is the corticosteroid residue of formula (II): 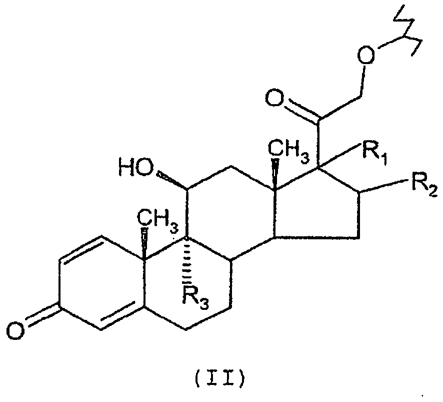 wherein
R₁ and R₂ both in position α are taken together and forms the group of formula (III) 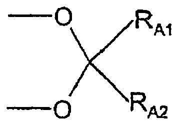 wherein R_{A1} and R_{A2} are -CH₃, ;
R₃ is a fluorine atom;
Z is a group capable of binding X selected from the group consisting of:
-C(O)-, -C(O)O- or 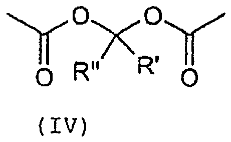 wherein R' and R" are independently selected from H or straight or branched C₁-C₄ alkyl;
X is a bivalent radical having the following meanings:
a) straight or branched C₁-C₂₀ alkylene, being optionally substituted with one or more of the substituents selected from the group consisting of: halogen atoms, hydroxy, -ONO₂ or T, wherein T is -OC(O) (C₁-C₁₀ alkyl)-ONO₂ or -O(C₁-C₁₀alkyl)-ONO₂;
b) a C₅-C₇ cycloalkylene group optionally substituted with linear or branched C₁-C₁₀ alkyl group;
c) 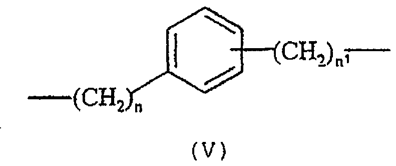
d) 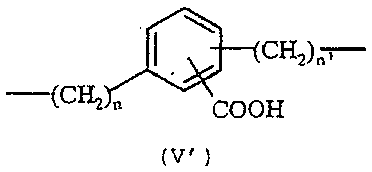 wherein n is an integer from 0 to 20;
n¹ is an integer from 1 to 20;
e) 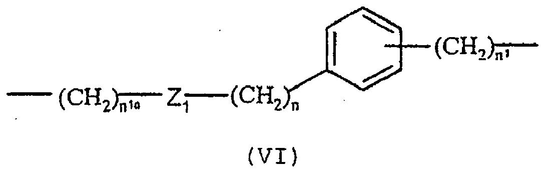 wherein n^{1a} is an integer from 1 to 20;
Z₁ is -C(O)O- or -OC(O)-;
n is as above defined;
n¹ is as above defined;
with the proviso that when X is selected from the bivalent radicals mentioned under c)-e), the -ONO₂ group of formula (I) is linked to the -(CH₂)ₙ¹- group;
f) 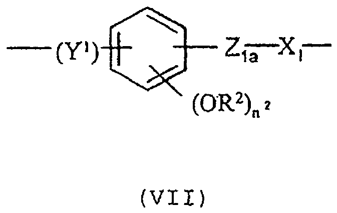 wherein:
Y¹ is -CH₂-CH₂-(CH₂)ₙ^{2a}-, or -CH=CH-(CH₂)ₙ^{2a}- wherein n^{2a} is an integer from 0 to 10;
Z₁ₐ is -OC (O) - or -C (O) O-;
n² is 0 or 1;
R² is H or CH₃;
X₁ is - (CH)ₙ^{1a}- wherein n^{1a} is as above defined, or the bivalent radical of formula (V) wherein n and n¹ are as above defined; with the proviso that in formula (VII) the -ONO₂ group of formula (I) is linked to the X₁ group;
g) 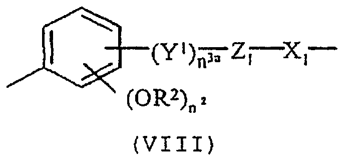 wherein:
Y¹ is -CH₂-CH₂-(CH₂)ₙ^{2a}-, or -CH=CH-(CH₂)ₙ^{2a}- wherein n^{2a} is an integer from 0 to 10;
n^{3a} is 0 or 1;
Z₁ is -C(O)O- or -OC(O)-;
n² is 0 or 1;
R² is H or CH₃;
X₁ is -(CH)ₙ^{1a}- wherein n^{1a} is as above defined, or the bivalent radical of formula (V) wherein n and n¹ are as above defined;
with the proviso that in formula (VIII) the -ONO₂ group of formula (I) is linked to the X₁ group;
h) 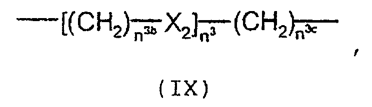 wherein
X₂ is -O- or -S-;
n³ is an integer from 1 to 6;
n^{3b} is an integer from 1 to 10;
n^{3c} is an integer from 1 to 10;
i) 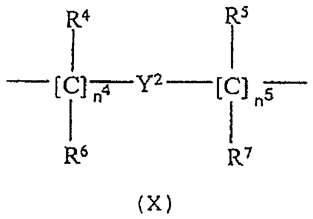 wherein:
n⁴ is an integer from 0 to 10;
n⁵ is an integer from 1 to 10;
R⁴, R⁵, R⁶, R⁷ are the same or different, and are H or straight or branched C₁-C₄ alkyl;
wherein the -ONO₂ group of formula (I) is linked to 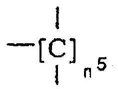
wherein n⁵ is as defined above;
Y² is an heterocyclic saturated, unsaturated or aromatic 5 or 6 membered ring, containing one or more heteroatoms selected from nitrogen, oxygen, sulfur,
and is selected from 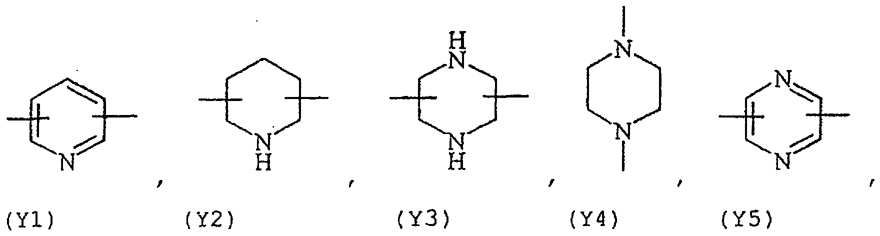 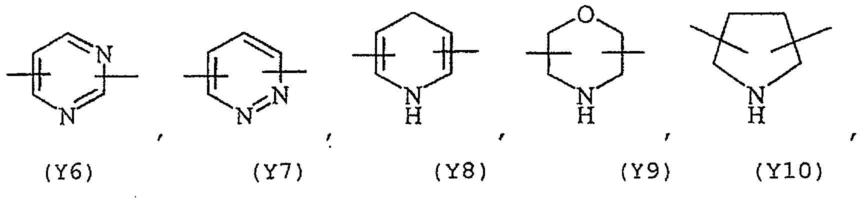 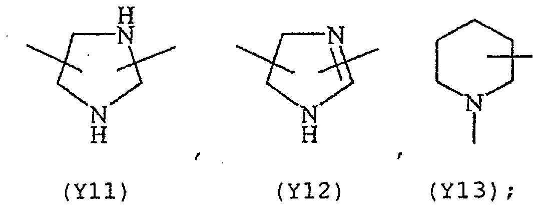
with the proviso that when in formula (I) Z is -C(O)- then X has not the following meaning:
a) straight or branched C₁-C₂₀ alkylene being optionally substituted with one or more of the substituents selected from the group consisting of: halogen atoms, hydroxy, -ONO₂ or T, wherein T is -OC(O)(C₁-C₁₀ alkyl)-ONO₂ or -O(C₁-C₁₀alkyl)-ONO₂.

2. Compounds of formula (I) according to claim 1 wherein
Z is -C(O)- or -C(O)O-;
X has the following meanings:
a) a straight or branched C₁-C₁₀ alkylene, being optionally substituted with one or more of the substituents selected from the group consisting of: halogen atoms, hydroxy, -ONO₂ or T, wherein T is -OC(O)(C₁-C₁₀ alkyl)-ONO₂ or -O(C₁-C₁₀alkyl)-ONO₂;
c) 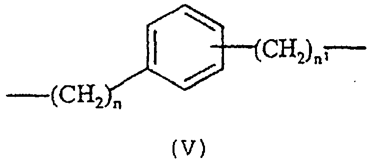
d) 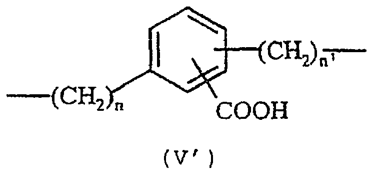 wherein n is an integer from 0 to 5;
n¹ is an integer from 1 to 5;
e) 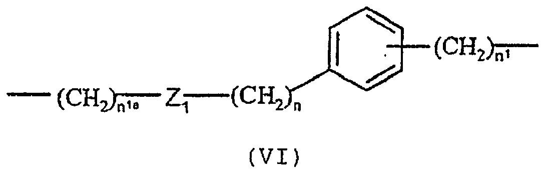 wherein n^{1a} is an integer from 1 to 10;
Z₁ is -C(O)O- or -OC(O)-;
n is as above defined;
n¹ is as above defined;
with the proviso that when X is selected from the bivalent radicals mentioned under c)-e), the -ONO₂ group of formula (I) is linked to the - (CH₂)ₙ¹- group;
f) 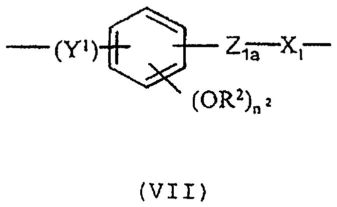 wherein:
Y¹ is -CH₂-CH₂-(CH₂)ₙ^{2a}-, or -CH=CH-(CH₂)ₙ^{2a}- wherein n^{2a} is 0 or is an integer from 1 to 6;
Z₁ₐ is -OC(O)- or -C(O)O-;
n² is 0 or 1;
R² is H or CH₃;
X₁ is - (CH)ₙ^{1a}- wherein n^{1a} is as above defined, or the bivalent radical of formula (V) wherein n and n¹ are as above defined;
with the proviso that in formula (VII) the -ONO₂ group of formula (I) is linked to the X₁ group;
g) 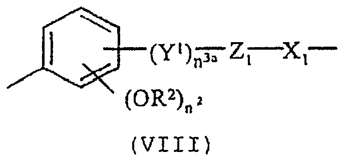 wherein:
Y¹ is -CH₂-CH₂-(CH₂)ₙ^{2a}-, or -CH=CH-(CH2)ₙ^{2a}- wherein n^{2a} is 0 or n^{2a} is an integer from 1 to 6;
n^{3a} is 0 or 1;
Z₁ is -C(O)O- or -OC(O)-;
n² is 0 or 1;
R² is H or CH₃;
X₁ is -(CH)ₙ^{1a}- wherein n^{1a}, is as above defined, or the bivalent radical of formula (V) wherein n and n¹ are as above defined; with the proviso that in formula (VIII) the -ONO₂ group of formula (I) is linked to the X₁ group;
h) 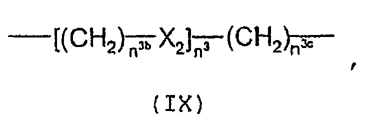 wherein
X₂ is -O- or -S-,
n³ is an integer from 1 to 6,
n^{3b} is an integer from 1 to 6,
n^{3c} is an integer from 1 to 6;
i) 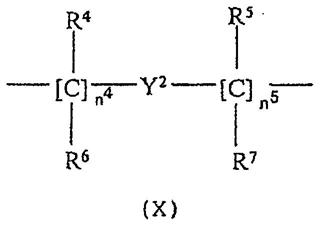 wherein:
n⁴ is an integer from 0 to 10;
n⁵ is an integer from 1 to 10;
R⁴, R⁵, R⁶, R⁷ are H;
wherein the -ONO₂ group of formula (I) is linked to 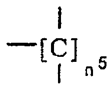
wherein n⁵ is as defined above;
Y² is an heterocyclic saturated, unsaturated or aromatic 5 or 6 membered ring, containing one or more heteroatoms selected from nitrogen, oxygen, sulfur, and is selected from 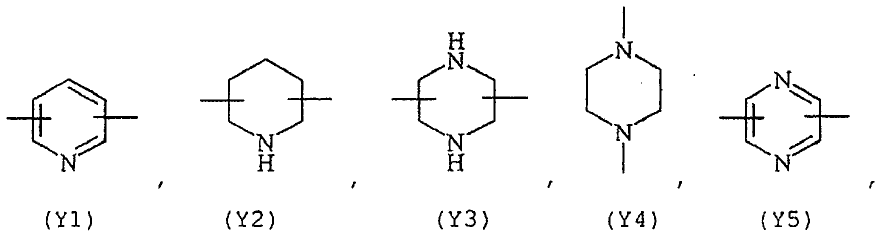 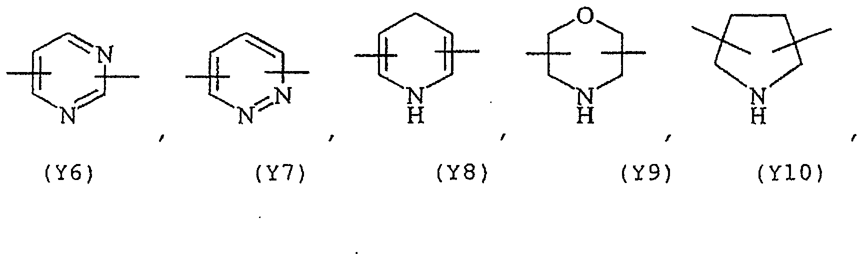 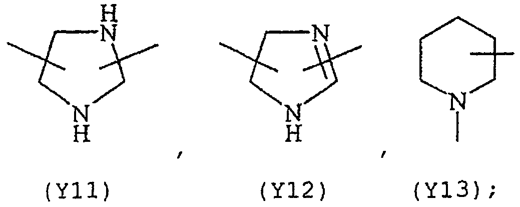
with the proviso that when in formula (I) Z is -C(O)-, then X has not the following meaning:
a) a straight or branched C₁-C₁₀ alkylene, being optionally substituted with one or more of the substituents selected from the group consisting of: halogen atoms, hydroxy, -ONO₂ or T, wherein T is -OC(O)(C₁-C₁₀ alkyl) -ONO₂ or -O(C₁-C₁₀ alkyl)-ONO₂

3. Compounds of formula (I) according to claim 2 wherein
X has the following meanings:
a) straight C₁-C₁₀ alkylene;
c) 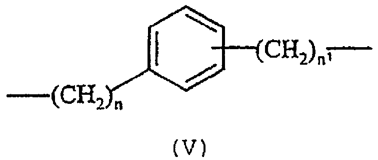 wherein n is 0 or 1 and n¹ is 1;
e) 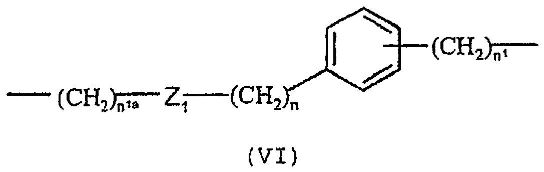 wherein n^{1a} is an integer from 1 to 10, Z₁ is -C(O)O- or -O(CO)-, n is 0 or 1 and n¹ is 1;
with the proviso that when X is selected from the bivalent radicals mentioned under c)-e), the -ONO₂ group of formula (I) is linked to the -(CH₂)ₙ¹- group;
f) 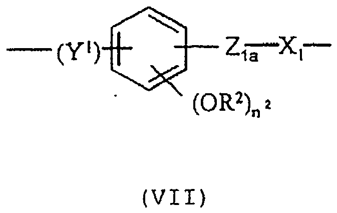 wherein:
Y¹ is -CH=CH-(CH₂)ₙ^{2a}- wherein n^{2a} is 0, Z₁ₐ is -OC(O)-, n² is 1, R² is CH₃, X₁ is -(CH)ₙ^{1a}-, , wherein n^{1a} is an integer from 1 to 10;
with the proviso that in formula (VII) the -ONO₂ group of formula (I) is linked to the X₁ group;
g) 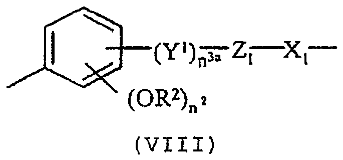 wherein:
n^{3a} is 1, Y¹ is -CH=CH-(CH₂)ₙ^{2a}- wherein n^{2a} is O, Z₁ is -C(O)O-, n² is 1, R² is CH₃, X₁ is -(CH)ₙ^{1a}- wherein n^{1a} is an integer from 1 to 10;
or in formula (VIII) n^{3a} is 0, Z₁ is -OC(O) - or -C(O)O-, n² is 1, R² is CH₃ and X₁ is -(CH)ₙ^{1a}- wherein n^{1a} is an integer from 1 to 10;
with the proviso that in formula (VIII) the -ONO₂ group of formula (I) is linked to the X₁ group;
h) 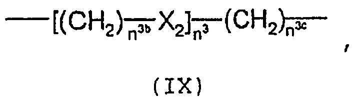 wherein
X₂ is -O-;
n³ is an integer from 1 to 4;
n^{3b} is 1;
n^{3c} is 2;
with the proviso that when in formula (I) Z is -C(O)-, then X can not be straight C₁-C₁₀ alkylene.

4. Compounds of formula (I) according to any of claims 1 to 3 selected from the following group:

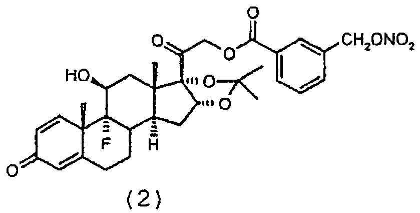

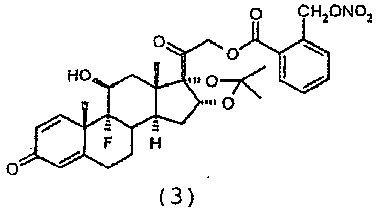

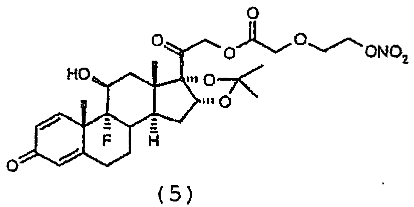

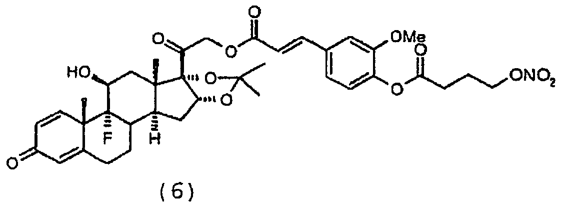

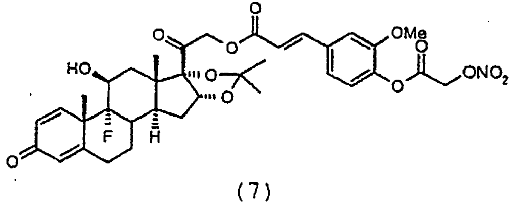

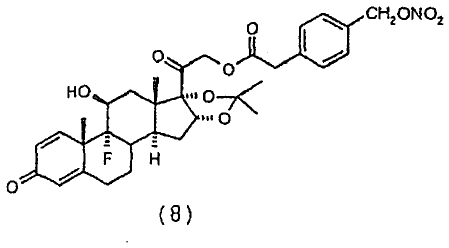

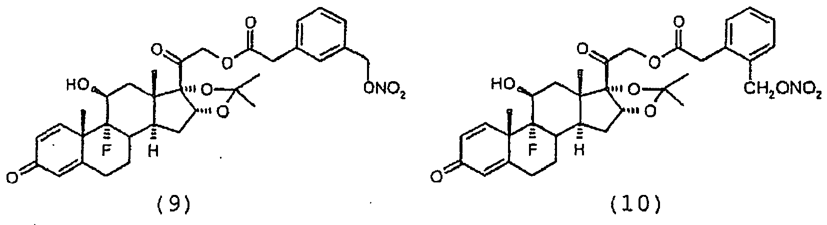

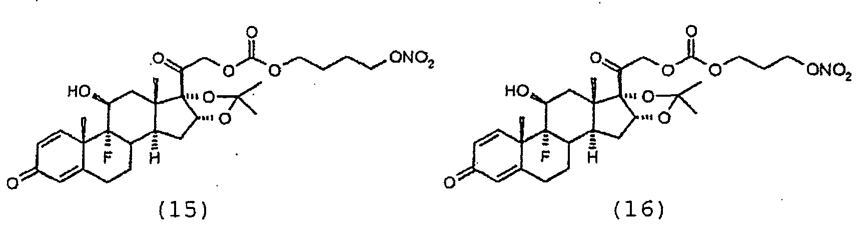

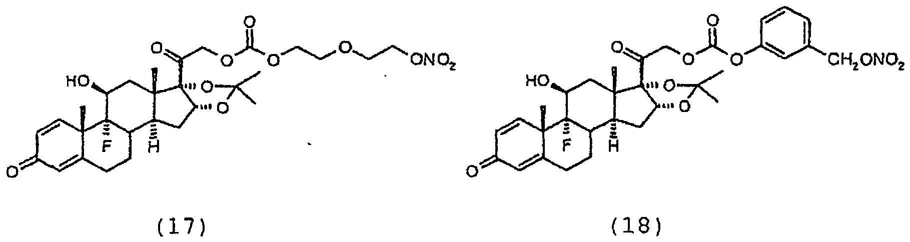

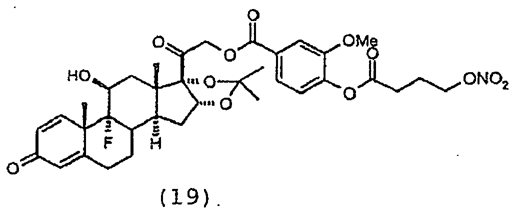

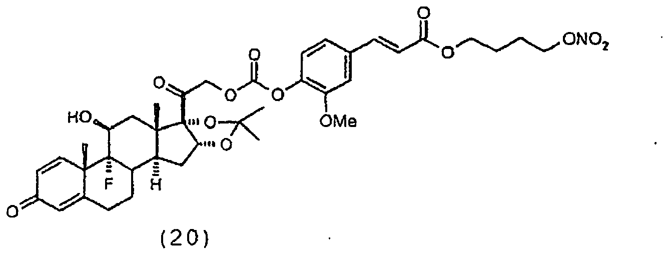

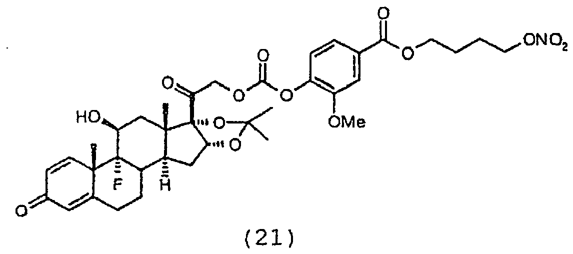

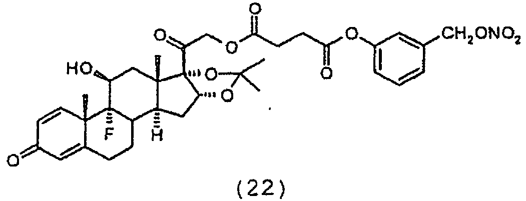

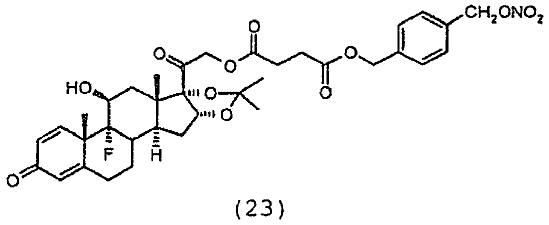

5. Compound of formula (I) according to claim 1 that is (11β,16α)-9-fluoro-11-hydroxy-16,17-[1-methylethylidenebis(oxy)]-21-[1-oxo-[4-(nitrooxymethyl)benzoxy]] pregna-1,4-diene-3,20-dione.

6. Compounds according to any of claims 1 to 5 for use as medicaments.

7. Use of the compounds of formula (I) according to claim 1 for the preparation of drugs for treating corticosteroid-responsive dermatosis, atopic dermatitis, contact dermatitis, psoriasis, seborrheic dermatitis.

8. Use of the compounds of formula (I) according to claim 1 for the preparation of drugs for treating skin diseases or disorders which comprise eczema, erythema, papulation, scaling, erosion, oozing, crusting, pruritis, inflammation, epidermolysis bullosa, erythema, warts, diaper rash, jock itch, lichen ruber planus .

9. Use of the compound of formula (I) of claim 5 for the preparation of drugs for treating corticosteroid-responsive dermatosis, inflammation, eczema, erythema, papulation, scaling, erosion, oozing, crusting, pruritis, epidermolysis bullosa, erythema, warts, diaper rash, jock itch, lichen ruber planus, seborrheic dermatitis.

10. Use of the compound of formula (I) of claim 5 for the preparation of drugs for treating atopic dermatitis.

11. Use of the compound of formula (I) of claim 5 for the preparation of drugs for treating contact dermatitis.

12. Use of the compound of formula (I) of claim 5 for the preparation of drugs for treating psoriasis.

13. Topical pharmaceutical formulation comprising at least a compound of formula (I) according to any of claims 1 to 4 and pharmaceutical acceptable excipients.

14. Topical pharmaceutical formulations according to claim 13 selected from the group comprising creams, lotions, ointment formulations or topical spray compositions.

15. Topical pharmaceutical formulation comprising the compound of formula (I) of claim 5 and pharmaceutical acceptable excipients.

16. Topical pharmaceutical formulations according to claim 15 selected from the group comprising creams, lotions, ointment formulations or topical spray compositions.

17. Process for the preparation of a compound of formula (I) of claim 1 wherein Z is -CO- comprising:
1a) reacting a compound of formula (IIa) 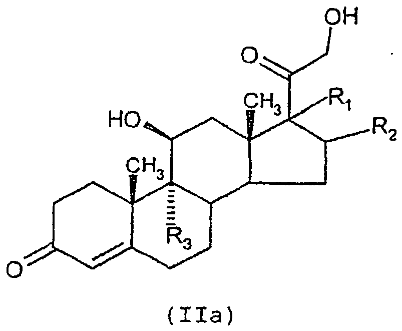 wherein
R₁ and R₂ when taken together are the group of formula (III) 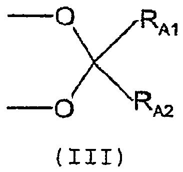 wherein R_{A1} and R_{A2} are CH₃;
R₁ and R₂ are linked to the carbon atoms in 16 and 17 of the steroidal structure in position α;
R₃ is a fluorine atom;
with a compound of formula (Ib)
W-C(O)-X-Q (Ib)
wherein
W is -OH, Cl, or -OC(O)Rₐ wherein Rₐ is a linear or branched C₁-C₅ alkyl or Rₐ is Rₐ₁ selected from the group consisting of: pentafluorphenoxy, 4-nitrophenoxy or succimidinyloxy;
X is as described in claim 1
Q is -ONO₂ or Z₂ wherein Z₂ is selected from the group consisting of: a chlorine atom, a bromine atom, a iodine atom, a mesyl group or a tosyl group, in the presence of a condensing agent, and
1b) when Q is Z₂, converting the compound obtained in the step a) into a nitro derivative by reaction with a nitrate source.

18. Process according to claim 17 wherein when W is -OH in the step 1a) the condensing agent is selected from the group comprising dicyclohexylcarbodiimide, N'-(3-dimethylaminopropyl)-N-ethylcarbodiimide hydrochloride and a catalyst, N,N-dimethylamino pyridine or N,N'-carbonyldiimidazole.

19. Process according to claim 17 wherein when W is -OC(O)Rₐ in the step 1a) the condensing agent is a catalyst selected from the group comprising: N,N-dimethylamino pyridine and a Lewis acid selected from Sc(OTf)₃ or Bi(OTf)₃.

20. Process according to claim 17 wherein when W is Cl and Q is Z₂, in the step 1a) the condensing agent is an organic base selected from the group comprising: N,N-dimethylamino pyridine, triethylamine or pyridine.

21. Process according to any of claims 17 to 20 wherein the step 1a) is carried out in a suitable organic solvent selected from N,N'-dimethylformamide, tetrahydrofuran, benzene, toluene, dioxane or a polyhalogenated aliphatic hydrocarbon, at a temperature from - 20°C and 40°C.

22. Process according to claim 17 wherein in step 1b) the source of nitrate is selected from the group comprising: silver nitrate, lithium nitrate, sodium nitrate, potassium nitrate, magnesium nitrate, calcium nitrate, iron nitrate, zinc nitrate or tetraalkylammonium nitrate.

23. Process according to claims 17 and 22 wherein the step 1b) is carried out in the dark, in a solvent selected from the group comprising: acetonitrile, tetrahydrofurane, methyl ethyl ketone, ethyl acetate or N,N'-dimethylformamide, at a temperature from room temperature to the boiling temperature of the solvent.

24. Process for the preparation of a compound of formula (I) of claim 1 wherein Z is -C(O)O- comprising:
2a) reacting a compound of formula (IIa) 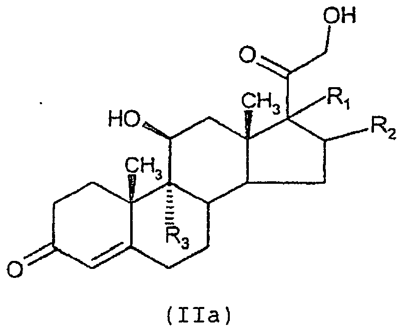 wherein
R₁ and R₂ when taken together are the group of formula (III) 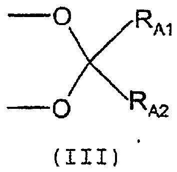 wherein R_{A1} and R_{A2} are CH₃;
R₁ and R₂ are linked to the carbon atoms in 16 and 17 of the steroidal structure in position α;
R₃ is a fluorine atom;
with a compound of formula (Ic)
R_{b}-C(O)O-X-Q (Ic)
wherein Q is -ONO₂ or Z₂ wherein Z₂ is selected from the group consisting of: a chlorine atom, a bromine atom, a iodine atom, a mesyl group or a tosyl group;
R_{b} is Cl, Br or Rₐ₁ wherein Rₐ₁ is selected from the group consisting of: pentafluorphenoxy, 4-nitrophenoxy or succimidinyloxy; X is as defined in claim 1
2b) when Q is Z₂, converting the compound obtained in the step 2a) into the nitro derivative by reaction with a nitrate source.

25. Process according to claim 24 wherein step 2a) is carried out in presence of a inorganic or organic base in an aprotic polar/non-polar solvent selected from the group comprising:
N,N'-dimethylformamide, tetrahydrofuran or a polyhalogenated aliphatic hydrocarbon, at temperatures in the range between 0°-65°C.

26. Process according to claim 24 wherein step 2a) is carried out in presence of an inorganic or organic base in a double phase system H₂O/Et₂O at temperatures in the range between 20°- 40°C.

27. Process according to claim 24 wherein step 2a) is carried out in presence of N,N-dimethylamino pyridine and a Lewis acid selected from Sc(OTf)₃ or Bi(OTf)₃, in a solvent selected from N,N'-dimethylformamide or a polyhalogenated aliphatic hydrocarbon.

28. Process according to claim 24 wherein in step 2b) the source of nitrate is selected from the group comprising: silver nitrate, lithium nitrate, sodium nitrate, potassium nitrate, magnesium nitrate, calcium nitrate, iron nitrate, zinc nitrate or tetraalkylammonium nitrate.

29. Process according to claims 24 and 28 wherein the step 2b) is carried out in the dark, in a solvent selected from the group comprising: acetonitrile, tetrahydrofurane, methyl ethyl ketone, ethyl acetate or N,N'-dimethylformamide, at a temperature from room temperature to the boiling temperature of the solvent.

## Patentansprüche

1. Verbindungen der allgemeinen Formel (I)
R-Z-X-ONO₂ (I)
wobei R der Corticosteroid-Rest der Formel (II) ist: 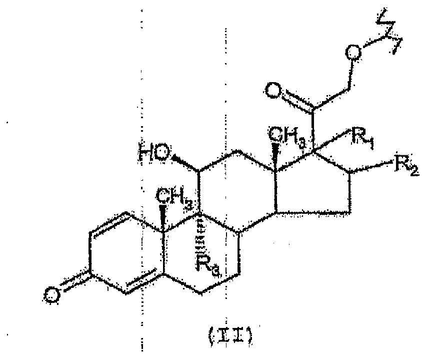 wobei:
R₁ und R₂, beide in Position α, zusammengenommen werden und den Rest der Formel (III) bilden 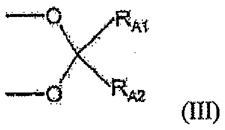
wobei R_{A1} und R_{A2} -CH₃ sind;
R₃ ein Fluoratom ist;
Z ein Rest ist, der in der Lage ist, X zu binden, ausgewählt aus der Gruppe bestehend aus:
-C(O)-, -C(O)O- oder 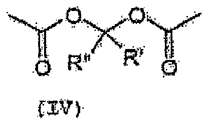
wobei R' und R" unabhängig ausgewählt sind aus H oder geradkettigen oder verzweigtem C₁-C₄-Alkyl;
X ein zweiwertiger Rest mit den folgenden Bedeutungen ist:
a) geradkettiges oder verzweigtes C₁-C₂₀-Alkylen, das gegebenenfalls mit einem oder mehreren der Substituenten, ausgewählt aus der Gruppe bestehend aus: Halogenatomen, Hydroxy, -ONO-₂ oder T, substituiert ist, wobei T -OC(O)(C₁-C₁₀-Alkyl)-ONO₂ oder -O(C₁-C₁₀-Alkyl)-ONO₂ ist;
b) ein C₅-C₇-Cycloalkylenrest, gegebenenfalls substituiert mit linearem oder verzweigtem C₁-C₁₀-Alkylrest;
c) 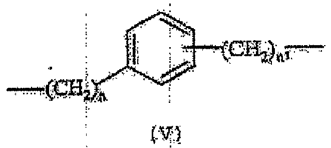
d) 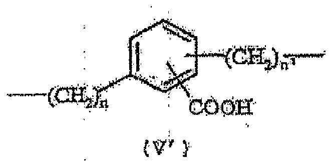 wobei n eine ganze Zahl von 0 bis 20 ist;
n¹ eine ganze Zahl von 1 bis 20 ist;
e) 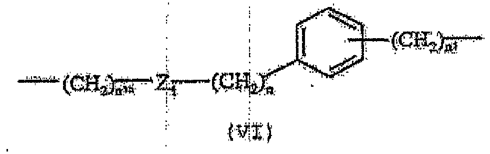 wobei n^{1a} eine ganze Zahl von 1 bis 20 ist;
Z₁ für -C(O)O- oder -OC(O)- steht;
n wie vorstehend definiert ist;
n¹ wie vorstehend definiert ist;
mit der Maßgabe, dass, wenn X aus den unter c)-e) genannten zweiwertigen Resten ausgewählt ist, der Rest -ONO₂ der Formel (I) an den Rest -(CH₂)ₙ¹- gebunden ist;
f) 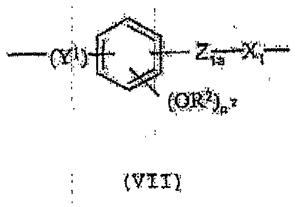 wobei:
Y¹ für -CH₂-CH₂-(CH₂)ₙ^{2a}- oder -CH=CH-(CH₂)ₙ^{2a}- steht, wobei n^{2a} eine ganze Zahl von 0 bis 10 ist;
Z₁ₐ für -OC(O)- oder -C(O)O- stellt;
n² 0 oder 1 ist;
R² für H oder CH₃ steht;
X₁ für -(CH)ₙ^{1a}-, wobei n^{1a} wie vorstehend definiert ist, oder den zweiwertigen Rest der Formal (V) steht, wobei n und n¹ wie vorstehend definiert sind;
mit der Maßgabe, dass in Formel (VII) der Rest -ONO₂ der Formel (I) an den Rest X₁ gebunden ist;
g) 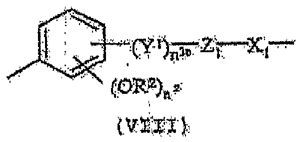 wobei:
Y¹ für -CH₂-CH₂-(CH₂)ₙ^{2a} oder -CH=CH-(CH₂)ₙ^{2a}- steht, wobei n^{2a} eine ganze Zahl von 0 bis 10 ist;
n^{3a} 0 oder 1 ist;
Z₁ -C(O)O- oder -OC(O)- ist;
n² 0 oder 1 ist;
R² für H oder CH₃ steht;
X₁ für -(CH)ₙ^{1a}-, wobei n^{1a} wie vorstehend definiert ist, oder den zweiwertigen Rest der Formel (V) steht, wobei n und n¹ wie vorstehend definiert sind;
mit der Maßgabe, dass in Formel (VIII) der Rest -ONO₂ der Formel (I) an den Rest X₁ gebunden ist;
h) 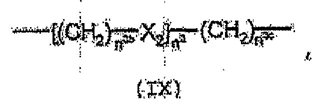 wobei
X₂ für -O- oder -S- steht;
n³ eine ganze Zahl von 1 bis 6 ist;
n^{3b} eine ganze Zahl von 1 bis 10 ist;
n^{3c} eine ganze Zahl von 1 bis 10 ist;
i) 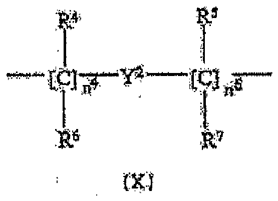 wobei:
n⁴ eine ganze Zahl von 0 bis 10 ist;
n⁵ eine ganze Zahl von 1 bis 10 ist;
R⁴, R⁵, R⁶, R⁷ gleich oder verschieden sind und H oder geradkettiges oder verzweigtes C₁-C₄-Alkyl sind;
wobei der Rest -ONO₂ der Formel (I) an 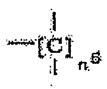 gebunden ist, wobei n⁵ wie vorstehend definiert ist;
Y² ein heterocyclischer gesättigter, ungesättigter oder aromatischer 5- oder 6-gliedriger Ring ist, der ein oder mehrere Heteroatome, ausgewählt aus Stickstoff, Sauerstoff, Schwefel, enthält,
und ausgewählt ist aus 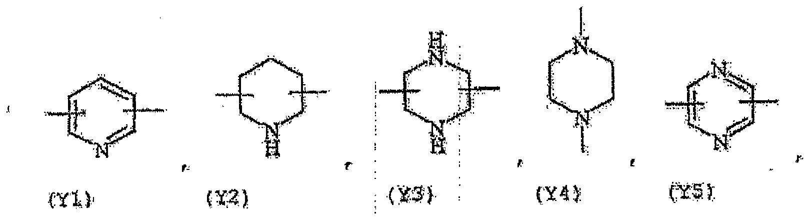 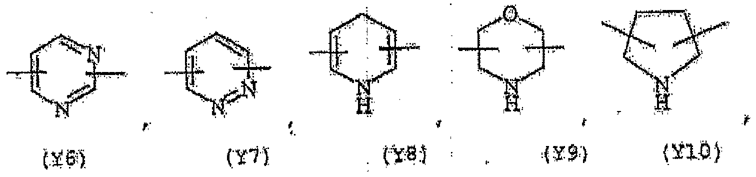 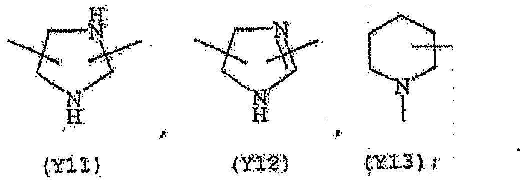 mit der Maßgabe, dass, wenn in Formel (I) Z gleich -C(O)- ist, dann X nicht die folgende Bedeutung hat:
a) geradkettiges oder verzweigtes C₁-C₂₀-Alkylen, das gegebenenfalls mit einem oder mehreren der Substituenten, ausgewählt aus der Gruppe bestehend aus: Halogenatomen, Hydroxy, -ONO₂ oder T, substituiert ist, wobei T für -OC(O)(C₁-C₁₀-Alkyl)-ONO₂ oder -O(C₁-C₁₀-Alkyl)-ONO₂ steht.

2. Verbindungen der Formel (I) nach Anspruch 1, wobei
Z für -C(O)- oder -C(O)O- steht;
X die folgenden Bedeutungen hat:
a) ein geradkettiges oder verzweigtes C₁-C₁₀-Alkylen, das gegebenenfalls mit einem oder mehreren der Substituenten, ausgewählt aus der Gruppe bestehend aus: Halogenatomen, Hydroxy, -ONO₂ oder T, substituiert ist, wobei T für -OC(O)(C₁-C₁₀-Alkyl)-ONO₂ oder -O(C₁-C₁₀-Alkyl)-ONO₂ steht;
c) 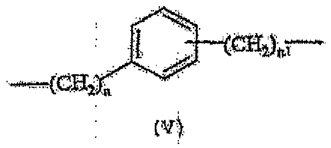
d) 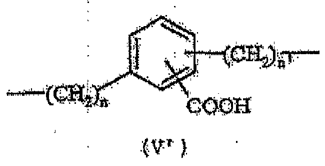 wobei n eine ganze Zahl von 0 bis 5 ist;
n¹ eine ganze Zahl von 1 bis 5 ist;
e) 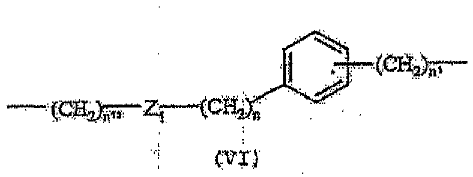 wobei n^{1a} eine ganze Zahl von 1 bis 10 ist;
Z₁ für -C(O)O- oder -OC(O)- steht;
n wie vorstehend definiert ist;
n¹ wie vorstehend definiert ist;
mit der Maßgabe, dass, wenn X aus den unter c)-e) genannten zweiwertigen Resten ausgewählt ist, der Rest -ONO₂ der Formel (I) an den Rest -(CH₂)ₙ¹- gebunden ist;
f) 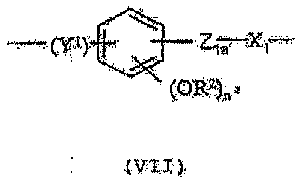 wobei:
Y¹ für -CH₂-CH₂-(CH₂)ₙ^{2a}- oder -CH=CH-(CH₂)ₙ^{2a}- steht, wobei n^{2a} 0 oder eine ganze Zahl von 1 bis 6 ist;
Z₁ₐ -OC(O)- oder -C(O)O- ist;
n² 0 oder 1 ist;
R² für H oder CH₃ steht;
X₁ für -(CH)ₙ^{1a}-, wobei n^{1a} wie vorstehend definiert ist, oder den zweiwertigen Rest der Formel (V) steht, wobei n und n¹ wie vorstehend definiert sind;
mit der Maßgabe, dass in Formel (VII) der Rest -ONO₂ der Formel (I) an den Rest X₁ gebunden ist;
g) 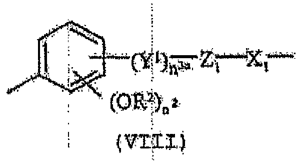 wobei:
Y¹ für -CH₂-CH₂-(CH₂)ₙ^{2a}- oder -CH=CH-(CH₂)ₙ^{2a}- steht, wobei n^{2a} 0 ist oder n^{2a} eine ganze Zahl von i bis 6 ist;
n^{3a} 0 oder 1 ist;
Z₁ für -C(O)O- der -OC(O)- steht;
n² 0 oder 1 ist;
R² für H oder CH₃ steht;
X₁ für -(CH)ₙ^{1a}-, wobei n^{1a} wie vorstehend definiert ist, oder den zweiwertigen Rest der Formel (V) steht, wobei n und n¹ wie vorstehend definiert sind;
mit der Maßgabe, dass in Formel (VIII) der Rest -ONO₂ der Formel (I) an den Rest X₁ gebunden ist;
h) 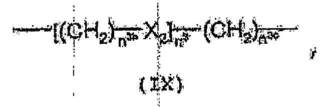 wobei
X₂ für -0- oder -S- steht;
n³ eine ganze Zahl von 1 bis 6 ist;
n^{3b} eine ganze Zahl von 1 bis 6 ist;
n^{3c} eine ganze Zahl von 1 bis 6 ist;
i) 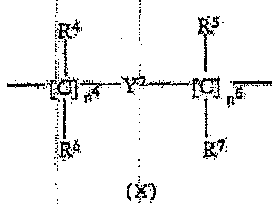 wobei:
n⁴ eine ganze Zahl von 0 bis 10 ist;
n⁵ eine ganze Zahl von 1 bis 10 ist;
R⁴, R⁵, R⁶, R⁷ gleich H sind;
wobei der Rest -ONO₂ der Formel (I) an 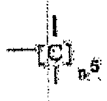 gebunden ist,
wobei n⁵ wie vorstehend definiert ist;
Y² ein heterocyclischer gesättigter, ungesättigter oder aromatischer 5- oder 6-gliedriger Ring ist, der ein oder mehrere Heteroatom, ausgewählt aus Stickstoff, Sauerstoff, Schwefel, enthält, und ausgewählt ist aus 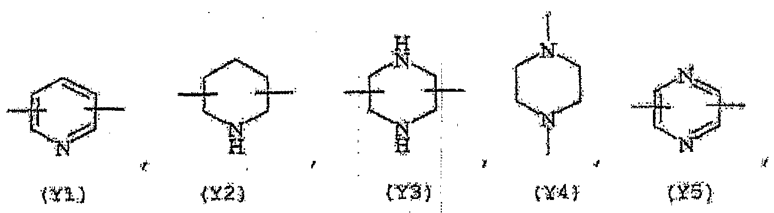 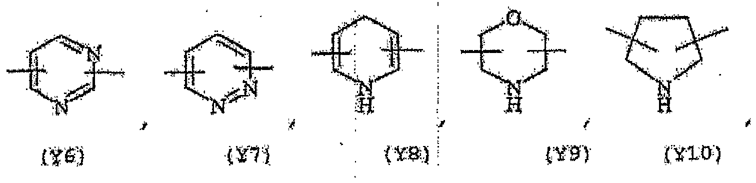 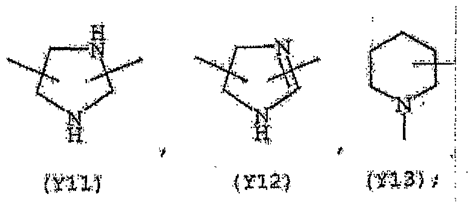
mit der Maßgabe, dass, wenn in Formel (I) Z gleich -C(O)- ist, dann X nicht die folgende Bedeutung hat:
a) ein geradkettiges oder verzweigtes C₁-C₁₀-Alkylen, das gegebenenfalls mit einem oder mehreren der Substituenten substituiert ist, ausgewählt aus der Gruppe bestehend aus: Halogenatomen, Hydroxy, -ONO₂ oder T, wobei T für -OC(O)(C₁-C₁₀-Alkyl)-ONO₂ oder -O(C₁-C₁₀-Alkyl)-ONO₂ steht.

3. Verbindungen der Formel (I) nach Anspruch 2, wobei X die folgenden Bedeutungen hat:
a) geradkettiges C₁-C₁₀-Alkylen;
c) 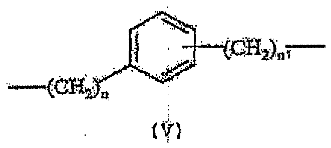 wobei n 0 oder 1 ist und n¹ ist;
e) 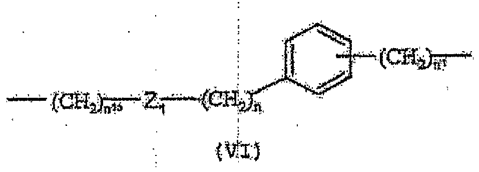 wobei n^{1a} eine ganze Zahl von 1 bis 10 ist, Z₁ für -C(O)O- oder -O(CO)- steht,
n 0 oder 1 ist und n¹ 1 ist;
mit der Maßgabe, dass, wenn X aus den unter c)-e) genannten zweiwertigen Resten ausgewählt ist, der Rest -ONO₂ der Formel (I) an den Rest -(CH₂)ₙ¹- gebunden ist;
f) 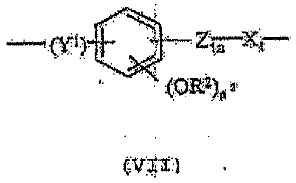 wobei:
Y¹ gleich -CH=CH-(CH₂)ₙ^{2a}- ist, wobei n^{2a} gleich 0 ist, Z₁ₐ -O(CO)- ist, n² 1 ist, R² gleich CH₃ ist, X₁ gleich -(CH)ₙ^{1a}- ist, wobei n^{1a} eine ganze Zahl von 1 bis 10 ist,
mit der Maßgabe, dass in Formel (VII) der Rest -ONO₂ der Formel (I) an den Rest X₁ gebunden ist;
g) 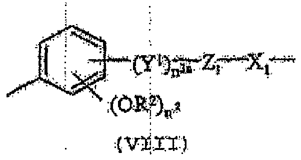 wobei:
n^{3a} 1 ist, Y¹ gleich -CH=CH-(CH₂)ₙ^{2a}- ist, wobei n^{2a} 0 ist, Z₁ gleich -C(O)O- ist, n² 1 ist, R² gleich CH₃ ist, X₁ gleich -(CH)ₙ^{1a}- ist, wobei n^{1a} eine ganze Zahl von 1 bis 10 ist;
oder in Formel (VIII) n^{3a} 0 ist, Z₁ gleich -OC(O)- oder -C(O)O- ist, n² 1 ist, R² gleich CH₃ ist und X₁ gleich -(CH)ₙ^{1a}- ist, wobei n^{1a} eine ganze Zahl von 1 bis 10 ist;
mit der Maßgabe, dass in Formel (VIII) der Rest -ONO₂ der Formel (I) an den Rest Xₜ gebunden ist;
h) 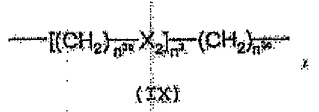 wobei
X₂ gleich -0- ist;
n³ eine ganze Zahl von 1 bis 4 ist;
n^{3b} 1 ist;
n^{3c} 2 ist;
mit der Maßgabe, dass, wenn in Formel (I) Z gleich -C(O)- ist, dann X nicht geradkettiges C₁-C₁₀-Alkylen sein kann.

4. Verbindungen der Formel (I) nach einem der Ansprüche 1 bis 3, ausgewählt aus der folgenden Gruppe:

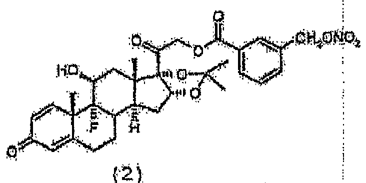

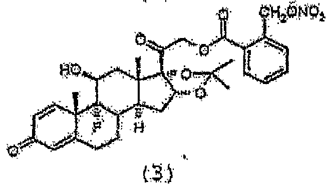

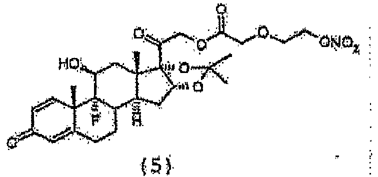

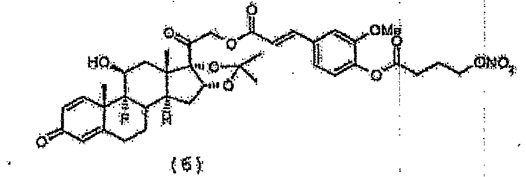

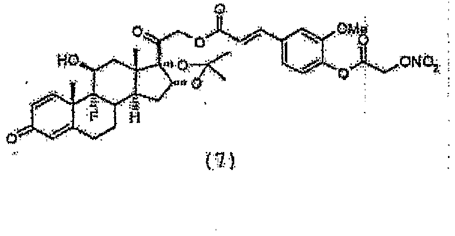

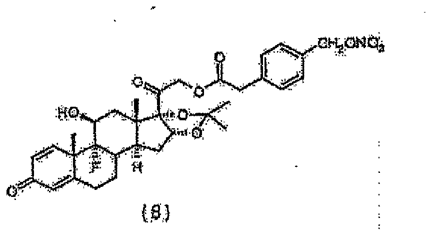

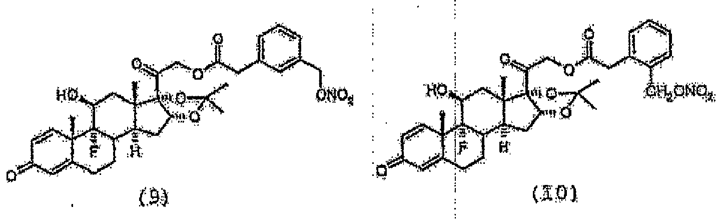

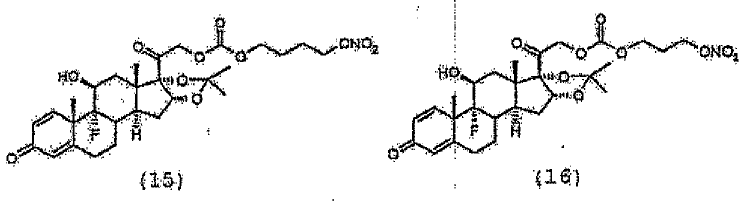

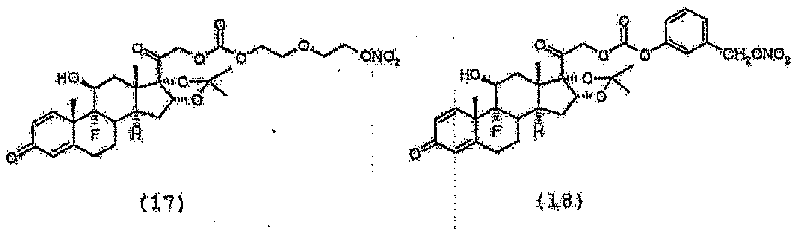

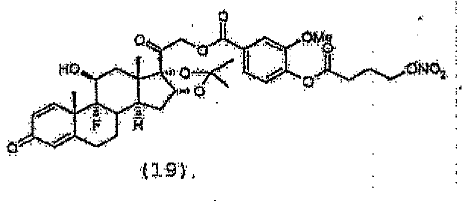

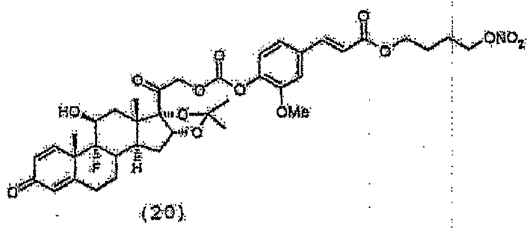

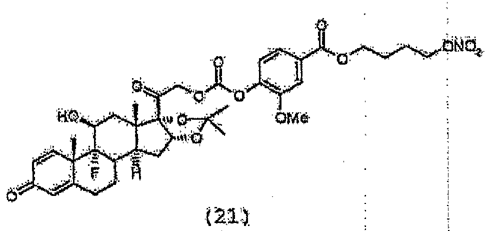

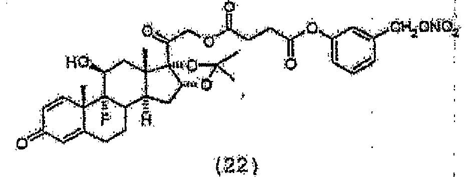

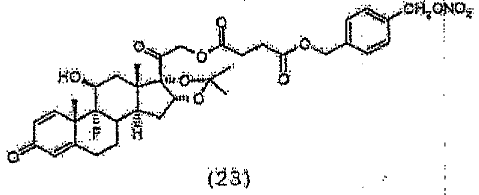

5. Verbindung der Formel (I) nach Anspruch 1, nämlich (11β,16α)-9-Fluor-11-hydroxy-16,17-[1-methylethylidenbis(oxy)]-21-[1-oxo-[4-(nitrooxymethyl)benzoxy]]pregna-1,4-dien-3,20-dion.

6. Verbindungen nach einem der Ansprüche 1 bis 5 zur Verwendung als Medikamente.

7. Verwendung der Verbindungen der Formel (I) nach Anspruch 1 zur Herstellung von Arzneimitteln zur Behandlung von auf Corticosteroid ansprechender Dermatose, atopischer Dermatitis, Kontaktdermatitis, Psoriasis, seborrhoischer Dermatitis.

8. Verwendung der Verbindungen der Formel (I) nach Anspruch 1 zur Herstellung von Arzneimitteln zur Behandlung von Hauterkrankungen oder -störungen, welche Ekzem, Erythem, Knötchenbildung, Schuppung, Erosion, Nässen, Verkrustung, Pruritus, Entzündung, Epidermolysis bullosa, Erythem, Warzen, Windeldermatitis, Tinea Cruris, Lichen ruber planus umfassen.

9. Verwendung der Verbindung der Formel (I) nach Anspruch 5 zur Herstellung von Arzneimitteln zur Behandlung von auf Corticosteroid ansprechender Dermatose, Entzündung, Ekzern, Erythem, Knötchenbildung, Schuppung, Erosion, Nässen, Verkrustung, Pruritus, Epidermolysis bullosa, Erythem, Warzen, Windeldermatitis, Tinea Cruris, Lichen ruber planus, seborrhoischer Dermatitis.

10. Verwendung der Verbindung der Formel (I) nach Anspruch 5 zur Herstellung von Arzneimitteln zur Behandlung atopischer Dermatitis.

11. Verwendung der Verbindung der Formel (I) nach Anspruch 5 zur Herstellung von Arzneimitteln zur Behandlung von Kontaktdermatitis.

12. Verwendung der Verbindung der Formel (I) nach Anspruch 5 zur Herstellung von Arzneimitteln zur Behandlung von Psoriasis.

13. Topische pharmazeutische Formulierung, umfassend mindestens eine Verbindung der Formel (I) nach einem der Ansprüche 1 bis 4 und pharmazeutisch verträgliche Exzipienten.

14. Topische pharmazeutische Formulierungen nach Anspruch 13, ausgewählt aus der Gruppe umfassend Cremes, Lotionen, Salbenformulierungen oder topische Sprayzusammensetzungen.

15. Topische pharmazeutische Formulierung, umfassend die Verbindung der Formel (I) nach Anspruch 5 und pharmazeutisch verträgliche Exzipienten.

16. Topische pharmazeutische Formulierungen nach Anspruch 15, ausgewählt aus der Gruppe umfassend Cremes, Lotionen, Salbenformulierungen oder topische Sprayzusammensetzungen.

17. Verfahren zur Herstellung einer Verbindung der Formel (I) nach Anspruch 1, wobei Z gleich -CO- ist, umfassend:
1a) Umsetzen einer Verbindung der Formel (IIa) 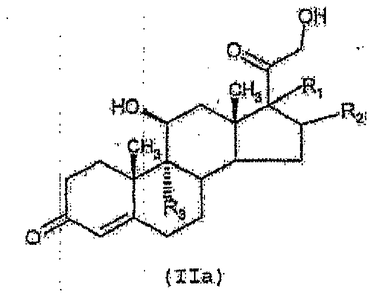 wobei
R₁ und R₂ zusammengenommen der Rest der Formel (III) sind 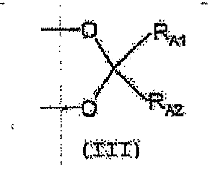 wobei R_{A1} und R_{A2} gleich CH₃ sind;
R₁ und R₂ an die Kohlenstoffatome in 16 und 17 der Steroidstruktur in Position α gebunden sind;
R₃ ein Fluoratom ist;
mit einer Verbindung der Formel (Ib)
W-C(O)-X-Q (Ib)
wobei
W für -OH, Cl oder -OC(O)Rₐ steht, wobei Rₐ ein lineares oder verzweigtes C₁-C₅-Alkyl ist oder Rₐ gleich Rₐ ist, ausgewählt aus der Gruppe bestehend aus:
Pentafluorphenoxy, 4-Nitrophenoxy oder Succimidinyloxy;
X wie in Anspruch 1 beschrieben ist;
Q gleich -ONO₂ oder Z₂ ist, wobei Z₂ ausgewählt ist aus der Gruppe bestehend aus: einem Chloratom, einem Bromatom, einem Iodatom, einer Mesylgruppe oder einer Tosylgruppe, in Gegenwart eines Kondensationsmittels und
1b) wenn Q gleich Z₂ ist, Umwandeln der in Schritt a) erhaltenen Verbindung in ein Nitroderivat durch Umsetzen mit einer Nitratquelle.

18. Verfahren nach Anspruch 17, wobei, wenn W gleich -OH ist, im Schritt 1a) das Kondensationsmittel ausgewählt ist aus der Gruppe, umfassend Dicyclohexylcarbodiimid, N'-(3-Dimethylaminopropyl)-N-ethylcarbodiimid-Hydrochlorid und einen Katalysator, N,N-Dimethylaminopyridin oder N,N'-Carbonyldiimidazol.

19. Verfahren nach Anspruch 17, wobei, wenn W gleich -OC(O)Rₐ ist, im Schritt 1a) das Kondensationsmittel ein Katalysator ist, ausgewählt aus der Gruppe, umfassend: N,N-Dimethylaminopyridin und eine Lewis-Säure, ausgewählt aus Sc(OTf)₃ oder Bi(OTf)₃.

20. Verfahren nach Anspruch 17, wobei, wenn W gleich Cl ist und Q gleich Z₂ ist, im Schritt 1a) das Kondensationsmittel eine organische Base ist, ausgewählt aus der Gruppe umfassend: N,N-Dimethylammopyridin, Trimethylamin oder Pyridin.

21. Verfahren nach einem der Ansprüche 17 bis 20, wobei der Schritt 1a) in einem geeigneten organischen Lösungsmittel, ausgewählt aus N,N'-Dimethylformamid, Tetrahydrofuran, Benzoyl, Toluol, Dioxan oder einem mehrfach halogenierten aliphatischen Kohlenwasserstoff, bei einer Temperatur von -20°C bis 40°C durchgeführt wird.

22. Verfahren nach Anspruch 17, wobei in Schritt 1b) die Nitratquelle ausgewählt ist aus der Gruppe, umfassend: Silbernitrat, Lithiumnitrat, Natriumnitrat, Kaliumnitrat, Magnesiumnitrat, Calciumnitrat, Eisennitrat, Zinknitrat oder Tetraalkylammoniumnitrat.

23. Verfahren nach den Ansprüchen 17 und 22, wobei der Schritt 1b) im Dunkeln, in einem Lösungsmittel, ausgewählt aus der Gruppe, umfassend: Acetonitril, Tetrahydrofuran, Methylethylketon, Ethylacetat oder N,N'-Dimethylformamid, bei einer Temperatur von Raumtemperatur bis zur Siedetemperatur des Lösungsmittels durchgeführt wird.

24. Verfahren zur Herstellung einer Verbindung der Formel (I) nach Anspruch 1, wobei Z gleich -C(O)O- ist, umfassend:
2a) Umsetzen einer Verbindung der Formel (IIa) 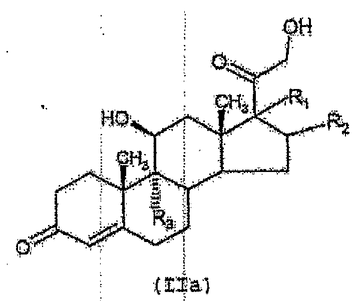 wobei
R₁ und R₂ zusammengenommen der Rest der Formel (III) sind 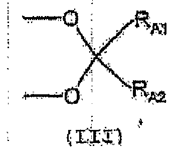 wobei R_{A} und R_{A2} gleich CH₃ sind;
R₁ und R₂ an die Kohlenstoffatome in 16 und 17 der Steroidstruktur in Position α gebunden sind;
R₃ ein Fluoratom ist;
mit einer Verbindung der Formel (Ic)
R_{b}=C(O)O-X-Q (Ic)
wobei Q gleich -ONO₂ oder Z₂ ist, wobei Z₂ ausgewählt ist aus der Gruppe bestehend aus: einem Chloratom, einem Bromatom, einem Iodatom, einer Mesylgruppe oder einer Tosylgruppe;
R_{b} gleich Cl, Br oder Rₐ₁ ist, wobei Rₐ₁ ausgewählt ist aus der Gruppe bestehend aus:
Pentafluorphenoxy, 4-Nitrophenoxy oder Succimidinyloxy; X wie in Anspruch 1 definiert ist;
2b) wenn Q gleich Z₂ ist, Umwandeln der im Schritt 2a) erhaltenen Verbindung in das Nitroderivat durch Umsetzen mit einer Nitratquelle.

25. Verfahren nach Anspruch 24, wobei Schritt 2a) in Gegenwart einer anorganischen oder organischen Base in einem aprotischen polaren/nicht-polaren Losungsmittel, ausgewählt aus der Gruppe, umfassend: N,N'-Dimethylformamid, Tetrahydrofuran oder einen mehrfach halogenierten aliphatischen Kohlenwasserstoff, bei Temperaturen im Bereich zwischen 0°-65°C durchgeführt wird.

26. Verfahren nach Anspruch 24, wobei Schritt 2a) in Gegenwart einer anorganischen oder organischen Base in einem Doppelphasensystem H₂O/Et₂O bei Temperaturen im Bereich zwischen 20°-40°C durchgeführt wird.

27. Verfahren nach Anspruch 24, wobei Schritt 2a) in Gegenwart von N,N-Dimethylaminopyridin und einer Lewissäure, ausgewählt aus Sc(OTf)₃ oder Bi(OTf)₃, in einem Lösungsmittel, ausgewählt aus N,N'-Dimethylformamid oder einem mehrfach halogenierten aliphatischen Kohlenwasserstoff, durchgeführt wird.

28. Verfahren nach Anspruch 24, wobei in Schritt 2b) die Nitratquelle ausgewählt ist aus der Gruppe, umfassend: Silbernitrat, Lithiumnitrat, Natriumnitrat, Kaliumnitrat, Magnesiumnitrat, Calciumnitrat, Eisennitrat, Zinknitrat oder Tetraalkylammoniumnitrat.

29. Verfahren nach den Ansprüchen 24 und 28, wobei der Schritt 2b) im Dunkeln, in einem Lösungsmittel, ausgewählt aus der Gruppe umfassend: Acetonitril, Tetrahydrofuran, Methylethylketon, Ethylacetat oder N,N'-Dimethylformamid, bei einer Temperatur von Raumtemperatur bis zur Siedetemperatur des Lösungsmittels durchgeführt wird.

## Revendications

1. Composés de formule générale (I)
R-Z-X-ONO₂ (I)
dans laquelle R est le résidu de corticostéroide de formule (II) : 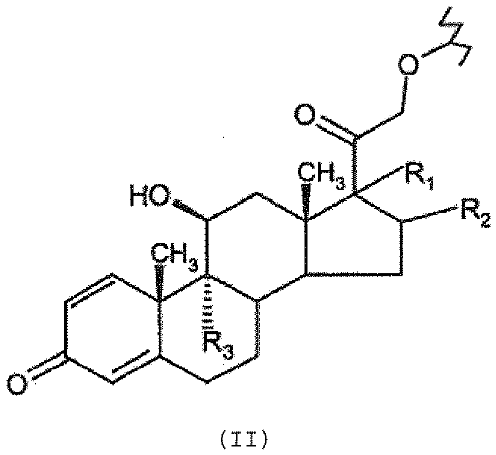 dans laquelle
R₁ et R₂, tous les deux en position α, sont pris ensemble et forment le groupe de formule (III) 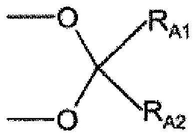 dans laquelle R_{A1} et R_{A2} sont -CH₃ ;
R₃ est un atome de fluor ;
Z est un groupe apte à lier X choisi parmi le groupe consistant en :
-C(O)-, -C(O)O- ou 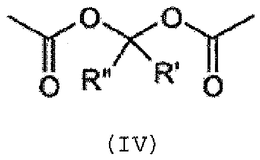 dans laquelle R' et R" sont indépendamment choisis parmi H ou un alkyle en C₁-C₄ linéaire ou ramifié ;
X est un radical bivalent ayant les sens suivants :
a) alkylène en C₁-C₂₀ linéaire ou ramifié, étant facultativement substitué avec un ou plusieurs des substituants choisi(s) parmi le groupe consistant en : des atomes d'halogène, hydroxy, -ONO₂ ou T, dans lequel T est - OC(O) (alkyle en C₁-C₁₀)-ONO₂ ou -O(alkyle en C₁-C₁₀)-ONO₂ ;
b) un groupe cycloalkylène en C₅-C₇ facultativement substitué avec un groupe alkyle en C₁-C₁₀ linéaire ou ramifié ;
c) 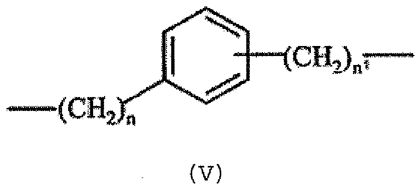
d) 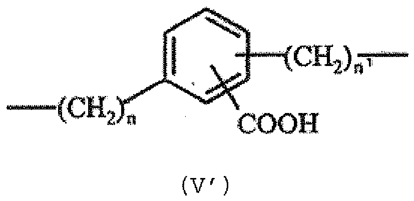 dans lesquelles n est un entier de 0 à 20 ;
n¹ est un entier de 1 à 20 ;
e) 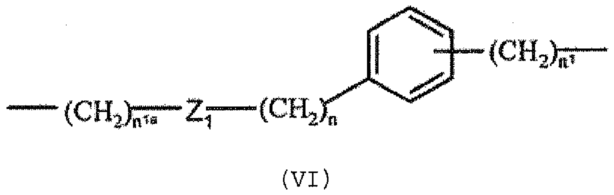 dans laquelle n^{1a} est un entier de 1 à 20 ;
Z₁ est -C(O)O- ou -OC(O)- ;
n est défini comme ci-dessus ;
n¹ est défini comme ci-dessus ;
sous réserve que, lorsque X est choisi parmi les radicaux bivalents mentionnés sous c)-e), le groupe -ONO₂ de formule (I) est lié au groupe -(CH₂)n¹- ;
f) 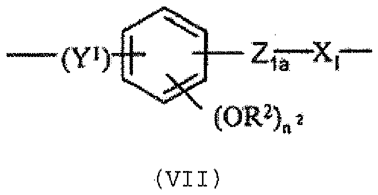 dans laquelle :
Y¹ est -CH₂-CH₂- (CH₂)ₙ^{2a}-, ou -CH=CH- (CH₂)ₙ^{2a}- dans lesquelles n^{2a} est un entier de 0 à 10 ;
Z₁ₐ est -OC(O)- ou -C(O)-O- ;
n² est 0 ou 1 ;
R² est H ou CH³ ;
X₁ est -(CH)ₙ^{1a}- dans laquelle n^{1a} est comme définie ci-dessus, ou le radical bivalent de formule (V) dans laquelle n et n¹ sont comme définis ci-dessus ;
sous réserve que, dans la formule (VII), le groupe -ONO₂ de formule (I) est lié au groupe X₁ ;
g) 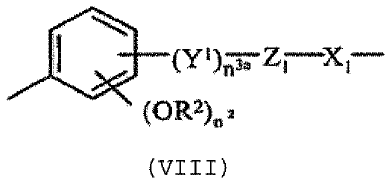 dans laquelle :
Y¹ est -CH₂-CH₂-(CH₂)ₙ^{2a}-, ou -CH=CH-(CH₂)ₙ^{2a}- dans lesquelles n^{2a} est un entier de 0 à 10 ;
n^{3a} est 0 ou 1 ;
Z₁ est -C(O)O- ou -OC(O)- ;
n² est 0 ou 1 ;
R² est H ou CH₃ ;
X₁ est -(CH)ₙ^{1a}- dans laquelle n^{1a} est comme définie ci-dessus, ou le radical bivalent de formule (V) dans laquelle n et n¹ sont comme définis ci-dessus ;
sous réserve que, dans la formule (VIII), le groupe -ONO₂ de formule (I) est lié au groupe X₁ ;
h) 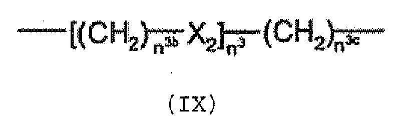 dans laquelle
X₂ est -O- ou -S- ;
n³ est un entier de 1 à 6 ;
n^{3b} est un entier de 1 à 10 ;
n^{3c} est un entier de 1 à 10 ;
i) 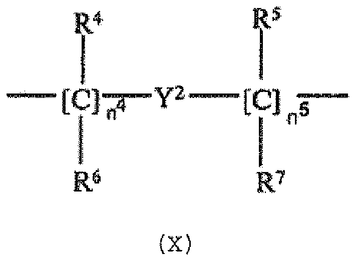 dans laquelle :
n⁴ est un entier de 0 à 10 ;
n⁵ est un entier de 1 à 10 ;
R⁴, R⁵, R⁶, R⁷ sont identiques ou différents, et sont H ou un alkyle en C₁-C₄ linéaire ou ramifié ;
dans laquelle le groupe -ONO₂ de formule (I) est lié à 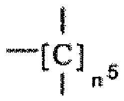
dans laquelle n⁵ est comme défini ci-dessus ;
Y² est un cycle à 5 ou 6 éléments hétérocyclique saturé, insaturé ou aromatique, contenant un ou plusieurs hétéroatome(s) choisi(s) parmi l'azote, l'oxygène, le soufre,
et est choisi parmi 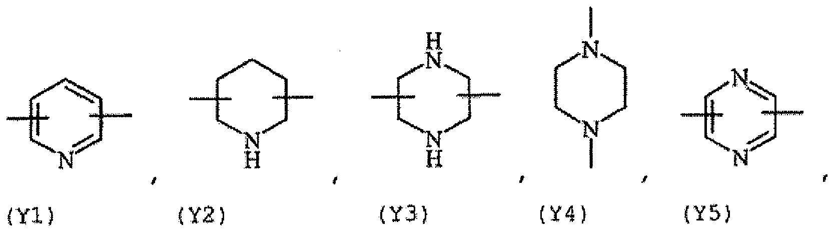 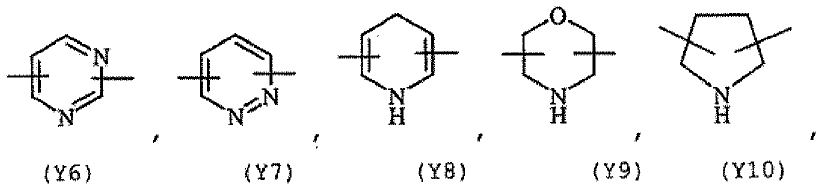 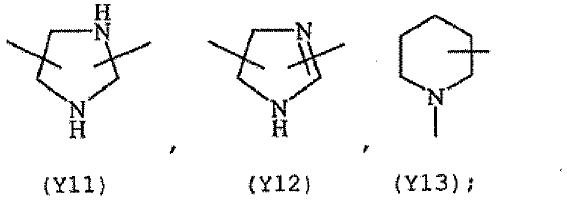 sous réserve que, lorsque dans la formule (I) Z est -C(O)-, alors X n'a pas le sens suivant :
a) alkylène en C₁-C₂₀ linéaire ou ramifié, étant facultativement substitué avec un ou plusieurs des substituants choisi(s) parmi le groupe consistant en : des atomes d'halogène, hydroxy, -ONO₂ ou T, dans lequel T est - OC(O) (alkyle en C₁-C₁₀) -ONO₂ ou -O(alkyle en C₁-C₁₀) -ONO₂.

2. Composés de formule (I) selon la revendication 1 dans lesquels
Z est -C(O)- ou -C(O)O-;
X a les sens suivants :
a) alkylène en C₁-C₂₀ linéaire ou ramifié, étant facultativement substitué avec un ou plusieurs des substituants choisi(s) parmi le groupe consistant en : des atomes d'halogène, hydroxy, -ONO₂ ou T, dans lequel T est - OC(O)(alkyle en C₁-C₁₀)-ONO₂ ou -O(alkyle en C₁-C₁₀)-ONO₂ ;
c) 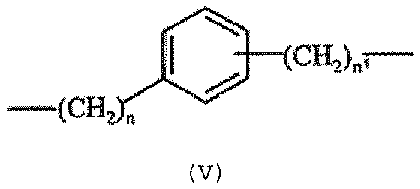
d) 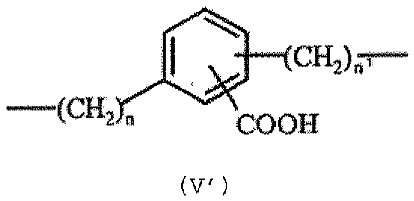 dans lesquelles n est un entier de 0 à 5 ;
n¹ est un entier de 1 à 5 ;
e) 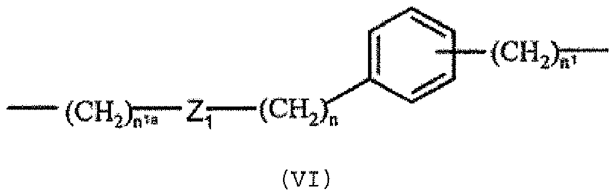 dans laquelle n^{1a} est un entier de 1 à 10 ;
Z₁ est -C(O)O- ou -OC(O)- ;
n est défini comme ci-dessus ;
n¹ est défini comme ci-dessus ;
sous réserve que, lorsque X est choisi parmi les radicaux bivalents mentionnés sous c)-e), le groupe -ONO₂ de formule (I) est lié au groupe -(CH₂)ₙ¹- ;
f) 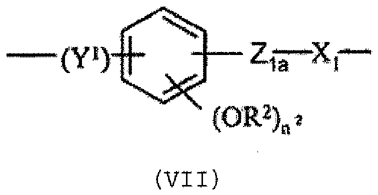 dans laquelle :
Y¹ est -CH₂-CH₂-(CH₂)ₙ^{2a}-, ou -CH=CH-(CH₂)ₙ^{2a}- dans lesquelles n^{2a} est 0 ou est un entier de 1 à 6 ;
Z₁ₐ est -OC(O)- ou -C(O)O-;
n² est 0 ou 1 ;
R² est H ou CH³ ;
X₁ est -(CH)ₙ^{1a}- dans laquelle n^{1a} est comme défini ci-dessus, ou le radical bivalent de formule (V) dans laquelle n et n¹ sont comme définis ci-dessus ;
sous réserve que, dans la formule (VII), le groupe -ONO₂ de formule (I) est lié au groupe X₁ ;
g) 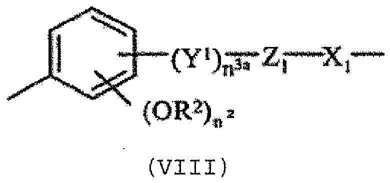 dans laquelle :
Y¹ est -CH₂-CH₂- (CH₂)ₙ^{2a}-, ou -CH=CH- (CH₂)ₙ^{2a}- dans lesquelles n^{2a} est 0 ou n^{2a} est un entier de 1 à 6 ;
n^{3a} est 0 ou 1 ;
Z₁ est -C(O)O- ou -OC(O)-;
n² est 0 ou 1 ;
R² est H ou CH₃ ;
X₁ est -(CH)ₙ^{1a}- dans laquelle n^{1a} est comme défini ci-dessus, ou le radical bivalent de formule (V) dans laquelle n et n¹ sont comme définis ci-dessus ;
sous réserve que, dans la formule (VIII), le groupe -ONO₂ de formule (I) est lié au groupe X₁ ;
h) 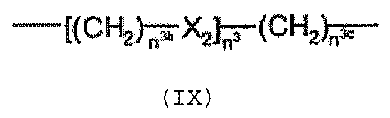 dans laquelle
X₂ est -O- ou -S- ;
n³ est un entier de 1 à 6 ;
n^{3b} est un entier de 1 à 6 ;
n^{3c} est un entier de 1 à 6 ;
i) 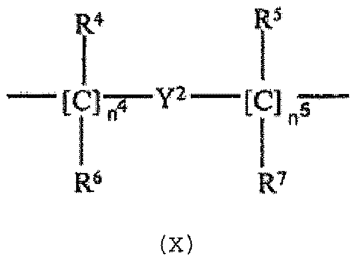 dans laquelle :
n⁴ est un entier de 0 à 10 ;
n⁵ est un entier de 1 à 10 ;
R⁴, R⁵, R⁶, R⁷ sont H ;
dans laquelle le groupe -ONO₂ de formule (I) est lié à 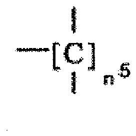
dans laquelle n⁵ est comme défini ci-dessus ;
Y² est un cycle à 5 ou 6 éléments hétérocyclique saturé, insaturé ou aromatique, contenant un ou plusieurs hétéroatome (s) choisi(s) parmi l'azote, l'oxygène, le soufre,
et est choisi parmi 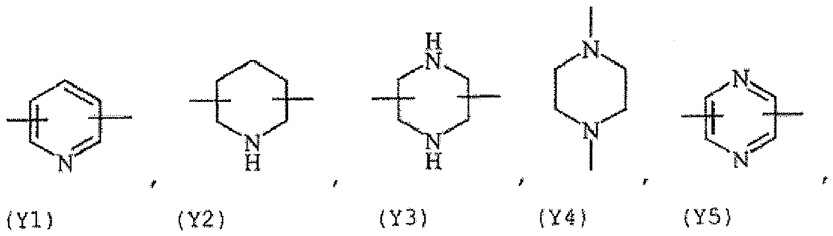 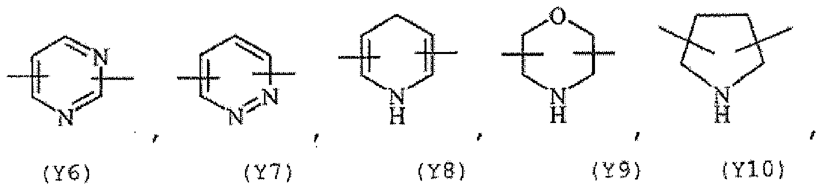 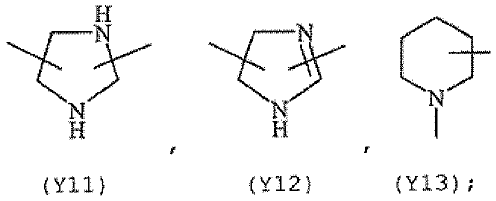
sous réserve que, lorsque dans la formule (I) Z est -C(O)-, alors X n'a pas le sens suivant :
a) alkylène en C₁-C₁₀ linéaire ou ramifié, étant facultativement substitué avec un ou plusieurs des substituants choisi(s) parmi le groupe consistant en : des atomes d'halogène, hydroxy, -ONO₂ ou T, dans lequel T est - OC(O)(alkyle en C₁-C₁₀)-ONO₂ ou -O(alkyle en C₁-C₁₀)-ONO₂.

3. Composés de formule (I) selon la revendication 2 dans lesquels
X a les sens suivants :
a) alkylène en C₁-C₁₀ linéaire ;
c) 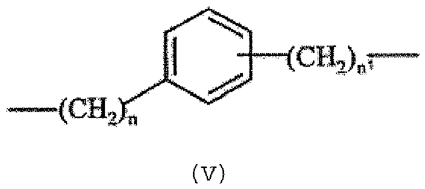 dans laquelle n est 0 ou 1 et n¹ est 1 ;
e) 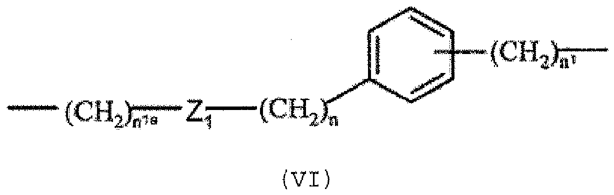 dans laquelle n^{1a} est un entier de 1 à 10, Z₁ est -C(O)O- ou -O(CO)-, n est 0 ou 1 et n¹ est 1 ;
sous réserve que, lorsque X est choisi parmi les radicaux bivalents mentionnés sous c)-e), le groupe -ONO₂ de formule (I) est lié au groupe -(CH₂)ₙ¹-;
f) 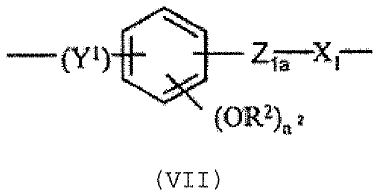 dans laquelle :
Y¹ est -CH=CH- (CH₂)ₙ^{2a}- dans laquelle n^{2a} est 0, Z₁ₐ est -OC(O)-, n² est 1, R² est CH³, X₁ est -(CH)ₙ^{1a}- dans laquelle n^{1a} est un entier de 1 à 10 ;
sous réserve que, dans la formule (VII), le groupe -ONO₂ de formule (I) est lié au groupe X₁ ;
g) 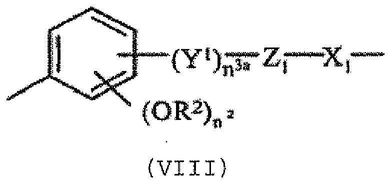 dans laquelle :
n^{3a} est 1, Y¹ est -CH=CH- (CH₂)ₙ^{2a}- dans laquelle n^{2a} est 0, Z₁ est -C(O)O-, n² est 1, R² est CH³, X₁ est -(CH)ₙ^{1a}- dans laquelle n^{1a} est un entier de 1 à 10 ;
ou, dans la formule (VIII), n^{3a} est 0, Z₁ est -OC(O) ou -C(O)O-, n² est 1, R² est CH₃, et X₁ est -(CH)ₙ^{1a}- dans laquelle n^{1a} est un entier de 1 à 10 ;
sous réserve que, dans la formule (VIII), le groupe -ONO₂ de formule (I) est lié au groupe X₁ ;
h) 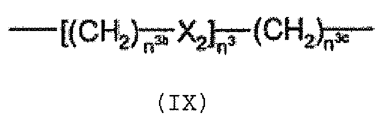 dans laquelle
X₂ est -O- ;
n³ est un entier de 1 à 4 ;
n^{3b} est 1 ;
n^{3c} est 2 ;
sous réserve que, lorsque dans la formule (I) Z est -C(O)-, alors X ne peut pas être un alkylène en C₁-C₁₀ linéaire.

4. Composés de formule (I) selon l'une quelconque des revendications 1 à 3 choisis parmi le groupe suivant :

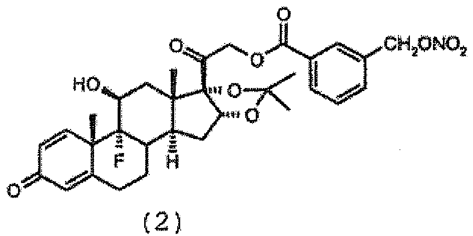

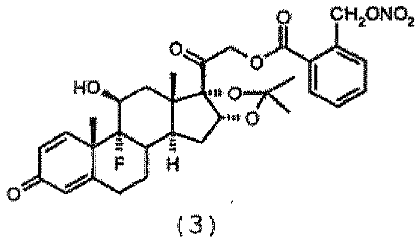

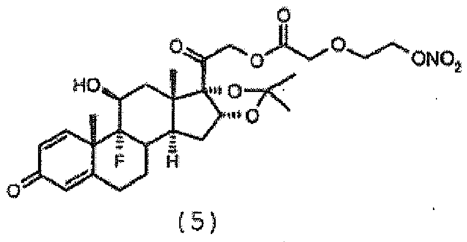

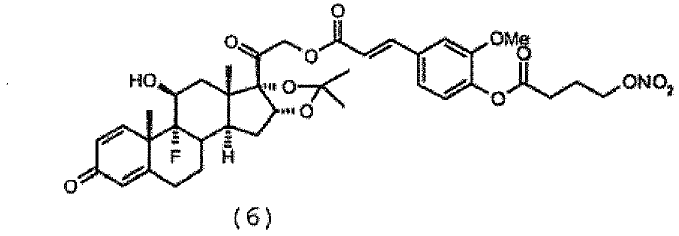

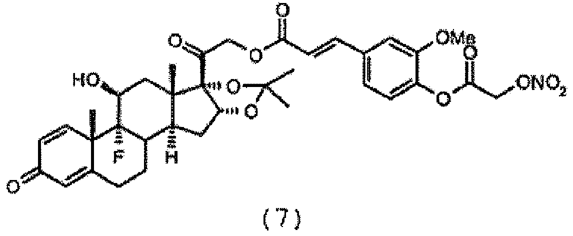

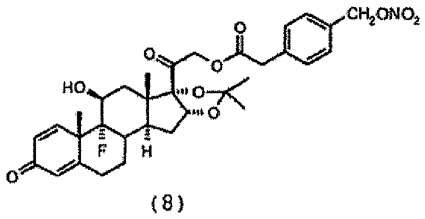

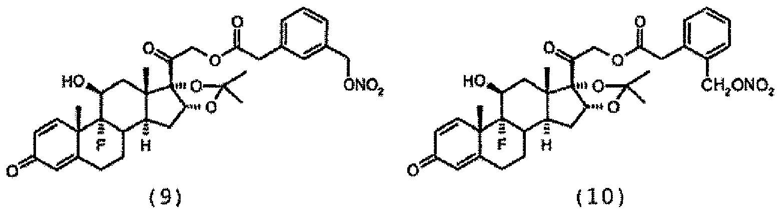

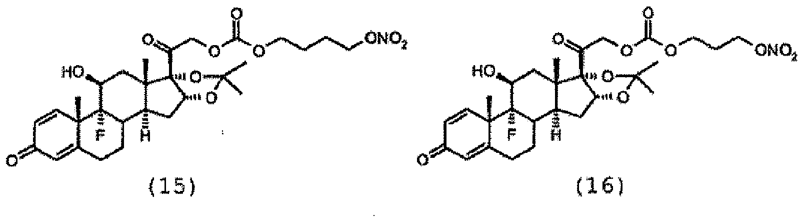

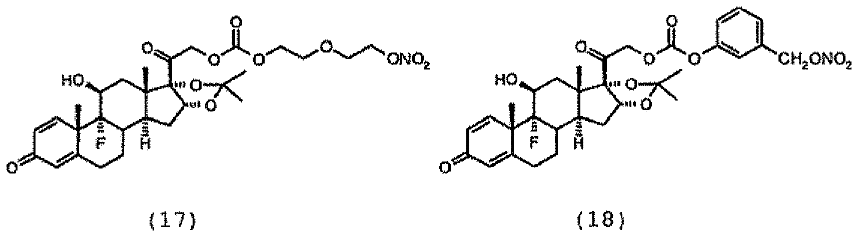

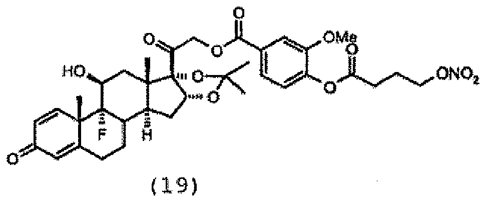

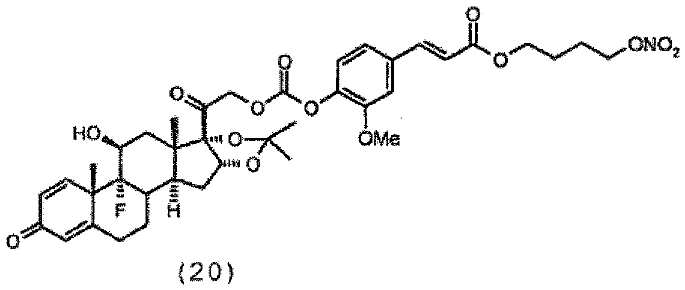

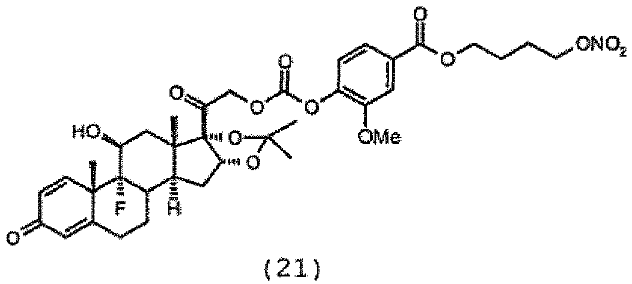

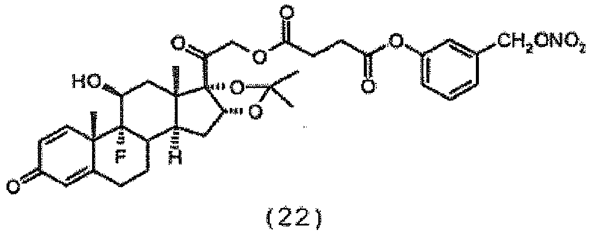

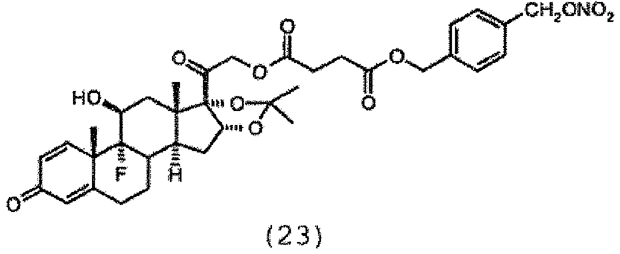

5. Composé de formule (I) selon la revendication 1 qui est la (11β,16α)-9-fluoro-11-hydroxy-16,17-[1-méthyléthylidènebis(oxy)]-21-{1-oxo-[4-(nitrooxyméthyl)benzoxy]}pregna-1,4-diène-3,20-dione.

6. Composés selon l'une quelconque des revendications 1 à 5 pour leur utilisation comme médicaments.

7. Utilisation des composés de formule (I) selon la revendication 1 pour la préparation de médicaments pour traiter une dermatose réagissant aux corticostéroïdes, une dermite atopique, une dermite de contact, un psoriasis, une dermite séborrhéique.

8. Utilisation des composés de formule (I) selon la revendication 1 pour la préparation de médicaments pour traiter des maladies ou des affections cutanées qui comprennent un eczéma, un érythème, une papulation, une desquamation, une érosion, un suintement, un croûtage, un prurit, une inflammation, une épidermolyse bulleuse, un érythème, des verrues, un érythème fessier, un eczéma marginé, un lichen plan.

9. Utilisation du composé de formule (I) selon la revendication 5 pour la préparation de médicaments pour traiter une dermatose réagissant aux corticostéroïdes, une inflammation, un eczéma, un érythème, une papulation, une desquamation, une érosion, un suintement, un croûtage, un prurit, une épidermolyse bulleuse, un érythème, des verrues, un érythème fessier, un eczéma marginé, un lichen plan, une dermite séborrhéique.

10. Utilisation du composé de formule (I) selon la revendication 5 pour la préparation de médicaments pour traiter une dermite atopique.

11. Utilisation du composé de formule (I) selon la revendication 5 pour la préparation de médicaments pour traiter une dermite de contact.

12. Utilisation du composé de formule (I) selon la revendication 5 pour la préparation de médicaments pour traiter un psoriasis.

13. Formulation pharmaceutique topique comprenant au moins un composé de formule (I) selon l'une quelconque des revendications 1 à 4 et des excipients pharmaceutiquement acceptables.

14. Formulations pharmaceutiques topiques selon la revendication 13 choisies parmi le groupe comprenant des crèmes, des lotions, des formulations d'onguents ou des compositions pour pulvérisations topiques.

15. Formulation pharmaceutique topique comprenant le composé de formule (I) selon la revendication 5 et des excipients pharmaceutiquement acceptables.

16. Formulations pharmaceutiques topiques selon la revendication 15 choisies parmi le groupe comprenant des crèmes, des lotions, des formulations d'onguents ou des compositions pour pulvérisations topiques.

17. Procédé pour la préparation d'un composé de formule (I) selon la revendication 1 dans laquelle Z est -CO- comprenant :
1a) la réaction d'un composé de formule (IIa) 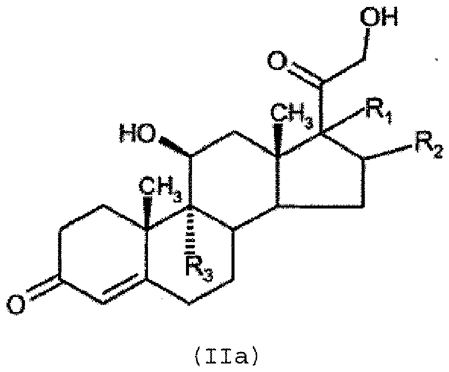 dans laquelle
R₁ et R₂, lorsqu'ils sont pris ensemble, sont le groupe de formule (III) 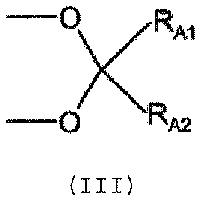 dans laquelle R_{A1} et R_{A2} sont CH₃ ;
R₁ et R₂ sont liés aux atomes de carbone en 16 et 17 de la structure stéroïdienne en position α ;
R₃ est un atome de fluor ;
avec un composé de formule (Ib)
W-C(O)-X-Q (Ib)
dans laquelle
W est -OH, Cl, ou -OC(O)Rₐ dans laquelle Rₐ est un alkyle en C₁-C₅ linéaire ou ramifié, ou bien Rₐ est Rₐ₁ choisi parmi le groupe consistant en : un pentafluorphénoxy, un 4-nitrophénoxy ou un succimidinyloxy ;
X est comme décrit dans la revendication 1
Q est -ONO₂ ou Z₂ dans lequel Z₂ est choisi parmi le groupe consistant en : un atome de chlore, un atome de brome, un atome d'iode, un groupe mésyle ou un groupe tosyle, en présence d'un agent de condensation, et
1b) lorsque Q est Z₂, la conversion du composé obtenu à l'étape a) en un dérivé nitro par réaction avec une source de nitrate.

18. Procédé selon la revendication 17 dans lequel, lorsque W est -OH à l'étape 1a), l'agent de condensation est choisi parmi le groupe comprenant le dicyclohexylcarbodiimide, le chlorhydrate de N'-(3-diméthylaminopropyl)-N-éthylcarbodiimide et un catalyseur, la N,N-diméthylaminopyridine ou le N,N'-carbonyldiimidazole.

19. Procédé selon la revendication 17 dans lequel, lorsque W est -OC(O)Rₐ à l'étape 1a), l'agent de condensation est un catalyseur choisi parmi le groupe comprenant : la N,N-diméthylaminopyridine et un acide de Lewis choisi parmi Sc(OTf)₃ ou Bi(OTf)₃.

20. Procédé selon la revendication 17 dans lequel, lorsque W est Cl et Q est Z₂, à l'étape 1a) l'agent de condensation est une base organique choisie parmi le groupe comprenant : la N,N-diméthylaminopyridine, la triéthylamine ou la pyridine.

21. Procédé selon l'une quelconque des revendications 17 à 20 dans lequel l'étape 1a) est mise en oeuvre dans un solvant organique approprié choisi parmi le N,N'-diméthylformamide, le tétrahydrofurane, le benzène, le toluène, le dioxane ou un hydrocarbure aliphatique polyhalogéné, à une température de -20°C à 40°C.

22. Procédé selon la revendication 17 dans lequel, à l'étape 1b), la source de nitrate est choisie parmi le groupe comprenant : le nitrate d'argent, le nitrate de lithium, le nitrate de sodium, le nitrate de potassium, le nitrate de magnésium, le nitrate de calcium, le nitrate de fer, le nitrate de zinc ou le nitrate de tétraalkylammonium.

23. Procédé selon les revendications 17 et 22 dans lequel l'étape 1b) est mise en oeuvre dans l'obscurité, dans un solvant choisi parmi le groupe comprenant : l'acétonitrile, le tétrahydrofurane, la méthyléthylcétone, l'acétate d'éthyle ou le N,N'-dimélhylformamide, à une température de la température ambiante à la température d'ébullition du solvant.

24. Procédé pour la préparation d'un composé de formule (I) selon la revendication 1 dans laquelle Z est -C(O)O- comprenant :
2a) la réaction d'un composé de formule (IIa) 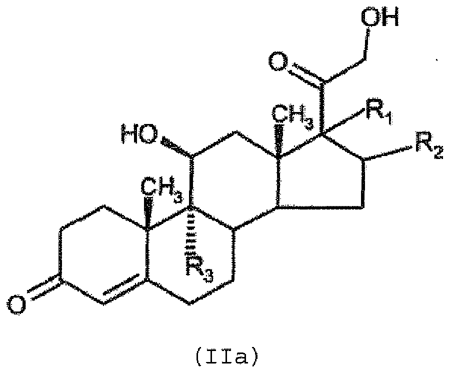 dans laquelle
R₁ et R₂, lorsqu'ils sont pris ensemble, sont le groupe de formule (III) 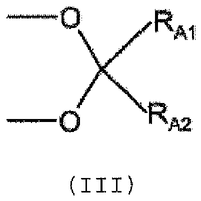 dans laquelle R_{A1} et R_{A2} sont CH₃ ;
R₁ et R₂ sont liés aux atomes de carbone en 16 et 17 de la structure stéroïdienne en position α ;
R₃ est un atome de fluor ;
avec un composé de formule (Ic)
R_{b}-C(O)O-X-Q (Ic)
dans laquelle Q est -ONO₂ ou Z₂ dans lequel Z₂ est choisi parmi le groupe consistant en : un atome de chlore, un atome de brome, un atome d'iode, un groupe mésyle ou un groupe tosyle ;
R_{b} est Cl, Br ou Rₐ₁ dans lequel Rₐ₁ est choisi parmi le groupe consistant en : un pentafluorphénoxy, un 4-nitrophénoxy ou un succimidinyloxy ;
X est comme défini dans la revendication 1 ;
2b) lorsque Q est Z₂, la conversion du composé obtenu à l'étape 2a) en un dérivé nitro par réaction avec une source de nitrate.

25. Procédé selon la revendication 24 dans lequel l'étape 2a) est mise en oeuvre en présence d'une base inorganique ou organique dans un solvant aprotique polaire/non polaire choisi parmi le groupe comprenant : le N,N'-diméthylformamide, le tétrahydrofurane ou un hydrocarbure aliphatique polyhalogéné, dans une plage de températures entre 0°C et 65°C.

26. Procédé selon la revendication 24 dans lequel l'étape 2a) est mise en oeuvre en présence d'une base inorganique ou organique dans un système à double phase H₂O/Et₂O dans une plage de températures entre 20°C et 40°C.

27. Procédé selon la revendication 24 dans lequel l'étape 2a) est mise en oeuvre en présence de N,N-diméthylaminopyridine et d'un acide de Lewis choisi parmi Sc(OTf)₃ ou Bi(OTf)₃, dans un solvant choisi parmi le N,N'-diméthylformamide ou un hydrocarbure aliphatique polyhalogéné.

28. Procédé selon la revendication 24 dans lequel, à l'étape 2b), la source de nitrate est choisie parmi le groupe comprenant : le nitrate d'argent, le nitrate de lithium, le nitrate de sodium, le nitrate de potassium, le nitrate de magnésium, le nitrate de calcium, le nitrate de fer, le nitrate de zinc ou le nitrate de tétraalkylammonium.

29. Procédé selon les revendications 24 et 28 dans lequel l'étape 2b) est mise en oeuvre dans l'obscurité, dans un solvant choisi parmi le groupe comprenant :
l'acétonitrile, le tétrahydrofurane, la méthyléthylcétone, l'acétate d'éthyle ou le N.N'-diméthylformamide, à une température de la température ambiante à la température d'ébullition du solvant.
